(19) Europäisches Patentamt
European Patent Office
Office européen des brevets

(11) Publication number : 0 322 391 B1

(12) # EUROPEAN PATENT SPECIFICATION

(45) Date of publication of patent specification : **25.03.92 Bulletin 92/13**

(51) Int. Cl.$^5$ : **A61K 31/135,** C07C 215/28, C07C 225/16, C07C 221/00

(21) Application number : **88850429.7**

(22) Date of filing : **20.12.88**

(54) **New phenylalkylamine derivatives.**

The file contains technical information submitted after the application was filed and not included in this specification

(30) Priority : **21.12.87 SE 8705089**
**25.11.88 SE 8804277**

(43) Date of publication of application : **28.06.89 Bulletin 89/26**

(45) Publication of the grant of the patent : **25.03.92 Bulletin 92/13**

(84) Designated Contracting States : **AT BE CH DE ES FR GB GR IT LI LU NL SE**

(56) References cited :
**US-A- 4 177 291**
**US-A- 4 469 707**
**Eur. J. Med. Chem.-Chimica Therapeutica November-December 1981-16, No 6, pages 495-501, U.H. Lindberg et al.:"Inhibitors of neuronal monoamine uptake III. Synthesis and pharmacologic screening of alaproclate analogues"**

(73) Proprietor : **AKTIEBOLAGET ASTRA**
**S-151 85 Södertälje (SE)**

(72) Inventor : **Gawell, Lars Ivar**
**Dammstugevägen 16B**
**S-155 00 Nykvarn (SE)**
Inventor : **Hallnemo, Gerd Margareta**
**Kämpevägen 39 III**
**S-151 54 Södertälje (SE)**
Inventor : **Högberg, Thomas**
**Batsmansvägen 28**
**S-151 39 Södertälje (SE)**
Inventor : **Lindberg, Ulf Henrik Anders**
**Kummelvägen 28**
**S-151 57 Södertälje (SE)**
Inventor : **Ström, Hans Eric Peter**
**Mossvägen 34**
**S-153 00 Järna (SE)**
Inventor : **Ulff, Carl Bengt Johan**
**Ägostigen 19**
**S-151 54 Södertälje (SE)**
Inventor : **Akesson, Christina Elizabeth**
**Kungsklippan 14 VI**
**S-112 25 Stockholm (SE)**
Inventor : **Ögren, Sven Ove**
**Hökmossvägen 14B**
**S-155 00 Nykvarn (SE)**

(74) Representative : **Linderoth, Margareta et al**
**AB Astra Patent Department**
**S-151 85 Södertälje (SE)**

EP 0 322 391 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The object of the present invention is to provide novel compounds and therapeutically acceptable salts thereof with a potentiating effect on cholinergic responses.

This invention also relates to processes for the preparation of the new compounds as well as to the pharmacological use of the new compounds and to pharmaceutical preparations containing such compounds.

### Prior art

US 4 237 311 describes some aralkyl esters of amino acids, e.g. alaproclate with a potential antidepressive activity combined with a reduced frequency of side effects. Alaproclate has the following structural formula

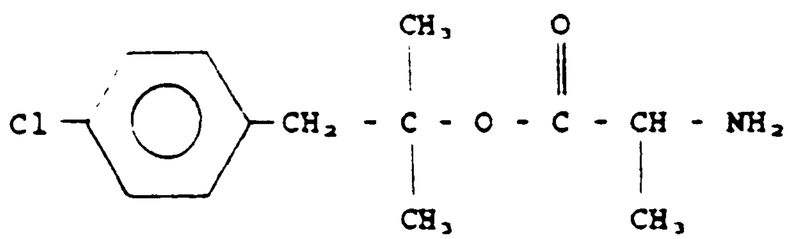

The compounds described in the above US patent exert their effect by blocking the neuronal 5-hydroxytryptamine (5-HT) uptake in the central nervous system. Some keto analogues of alaproclate are described by Lindberg U.H. et al. in European Journal of Medicinal Chemistry 16, 495-501 (1981). All compounds described in the cited paper, and containing a terminal primary amino group showed some degree of potency in inhibiting the neuronal uptake of 5-HT in vitro.

US 4 177 291 discloses some aminoketones of the general formula

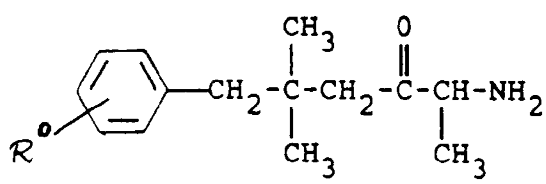

wherein

$R^0$ is selected from the group consisting of hydrogen, chlorine, bromine, methyl, trifluoromethyl and methoxy,

having effect on the central nervous system, especially antidepressive activity.

Furthermore it is known from US 4 469 707 that certain compounds, including alaproclate, described in US 4 237 311 besides from being inhibitors of the 5-HT uptake also have a potentiating effect on cholinergic responses.

### Outline of the invention

According to the present invention it has been found that the new compounds of the general formula I,

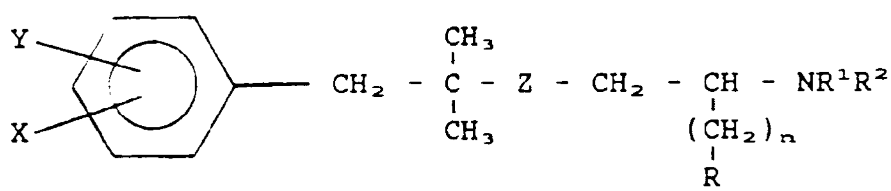

I

wherein n is 0 or 1, Z is CO or CH(OH), $R^1$ and $R^2$ are equal or different and, when equal, hydrogen, methyl, ethyl or propyl and, when different, $R^1$ is hydrogen and $R^2$ is methyl, ethyl, propyl or isopropyl, X and Y represent hydrogen, halogen, $CF_3$, CN or an alkyl group, straight or branched having 1-3 carbon atoms, in the ortho, meta

or para position provided that when X is in the ortho position and other than hydrogen then Y is other than hydrogen, and when Y is in the ortho position and other than hydrogen then X is other than hydrogen, R represents a straight or branched saturated or unsaturated 1-6 carbon alkyl group, a 3-6 carbon cycloalkyl group, a phenyl group, which may be unsubstituted or may carry a ring substituent $X^1$, $X^1$ preferably being a halogen, or R represents hydrogen, in racemic or optically active form and when Z is CH(OH) also mixtures of diastereomers or a pure diastereomer, with the proviso that the racemate of the compound wherein $R^1$ and $R^2$ is hydrogen, X is p-Cl, Y is hydrogen, Z is CO, n is 0 and R is methyl is excluded, or pharmaceutically acceptable salts thereof, have valuable pharmacological properties. Preferably $R^1$ and/or $R^2$ is hydrogen or methyl and most preferred $R^1$ and $R^2$ is hydrogen.

R is, when alkyl, preferably saturated straight or branched having 1-3 carbon atoms in the chain.

R has, when cycloalkyl, preferably 5-6 carbon atoms in the ring.

X, Y and $X^1$ are preferably in the meta and/or para positions.

Halogen X, Y and $X^1$ is preferably bromo, chloro or fluoro in the meta and/or para positions and in particular in the para position when being the monosubstituent. Most preferred is para fluoro.

When X and/or Y is $CF_3$ it is preferably in the meta position when being the monosubstituent, and in particular when R is a saturated alkyl group straight or branched having 1-3 carbon atoms in the chain.

When X and/or Y is CN it is preferably in the para position when being the monosubstituent, and in particular when R is a saturated alkyl group straight or branched having 1-3 carbon atoms in the chain.

When X and/or Y is alkyl it is preferably methyl in the para position when being the monosubstituent, and in particular when R is a saturated alkyl group straight or branched having 1-3 carbon atoms in the chain.

In a limited aspect the invention relates to new compounds of the general formula Ia,

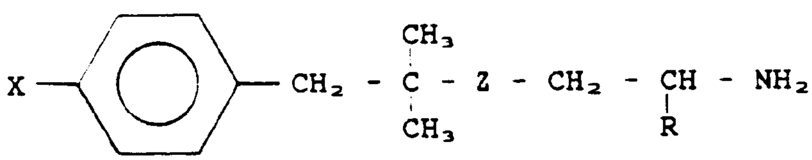

Ia

wherein Z is CO or CH(OH), X is halogen and R is a straight or branched alkyl group having 1-5 carbon atoms in the chain in racemic or optically active form, and when Z is CH(OH) also mixtures of diastereomers or the pure diastereomers, with the proviso that the racemate of the compound, wherein X is Cl, Z is CO and R is methyl is excluded;

or pharmaceutically acceptable salts thereof.

In the compounds of the general formula Ia halogen is preferably chloro or fluoro and R is preferably an ethyl or isopropyl group.

Most preferred among compounds of formula I is the following compound:

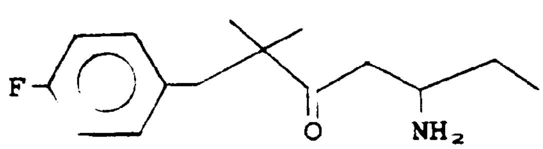

(Example 1)

as the racemate and as the pure (+) and (-) form.

Further preferred among compounds of formula I are the following compounds:

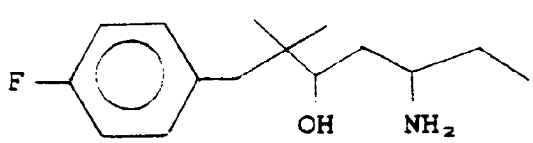

(Example 7)

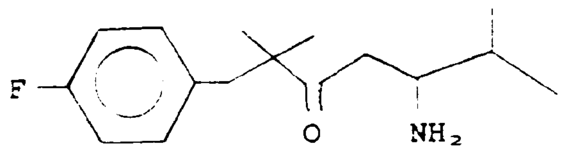

Example 12

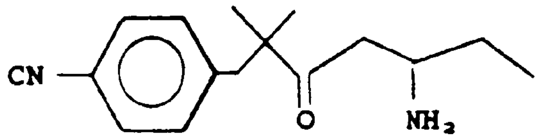

(Example 25)

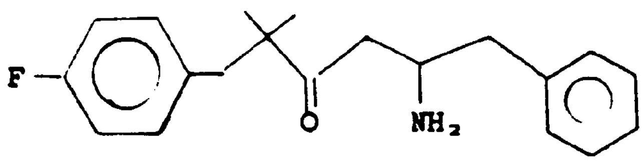

(Example 22)

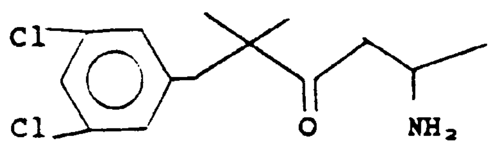

(Example 21)

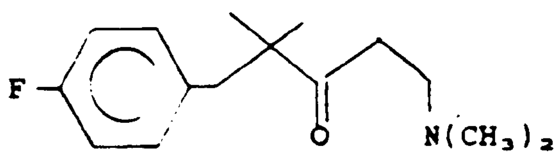

(Example 27)

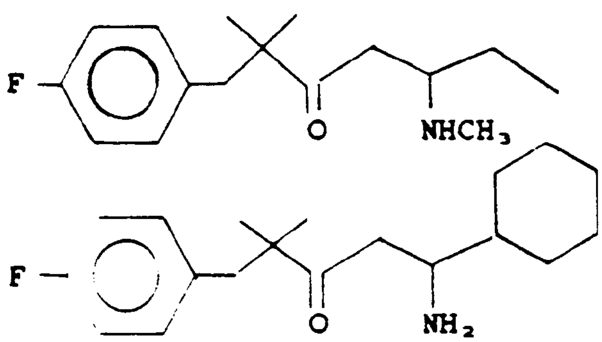

(Example 29)

(Example 24)

The compounds of the invention potentiate transmittor responses, probably by the modulation of ion-channels coupled to various neurotransmittor receptors. These effects make it possible to use the compounds in the treatment of mental disturbances eg psychosis, schizophrenia, schizoaffective conditions, anxiety, unipolar and bipolar depression as well as in the treatment of minimal memory impairment (MMI) and of senile dementia of the Alzheimer type (SDAT) In particular, certain compounds have a potentiating effect on central cholinergic responses. This potentiation is probably mediated by effects on ion channels, most likely of the potassium type. The latter two disturbances, MMI and SDAT, are believed to be associated with cholinergic dysfunction.

**Preparation**

The compounds of the general formula I

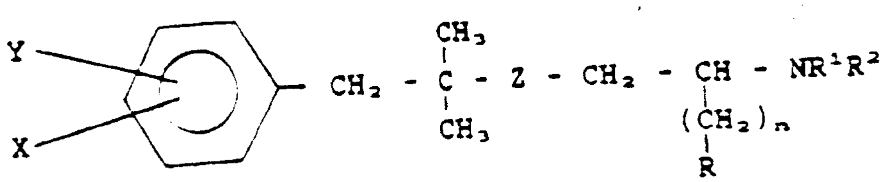 I

wherein Z, X, Y, R, $R^1$, $R^2$ and n are as defined above are prepared by

A) reacting a compound of the formula II

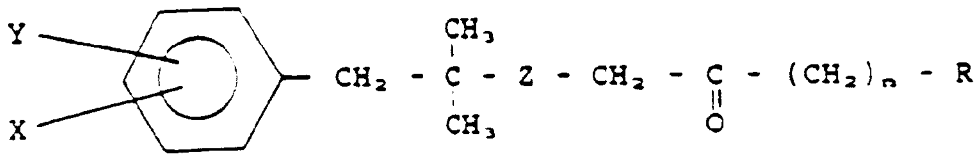 II

wherein Z, X, Y, R, and n are as defined above, with ammonia, an ammonium salt or a suitable amine derivative in the presence of a suitable reducing agent in a stepwise or direct manner, or

B) reduction of the corresponding derivative of the formula III

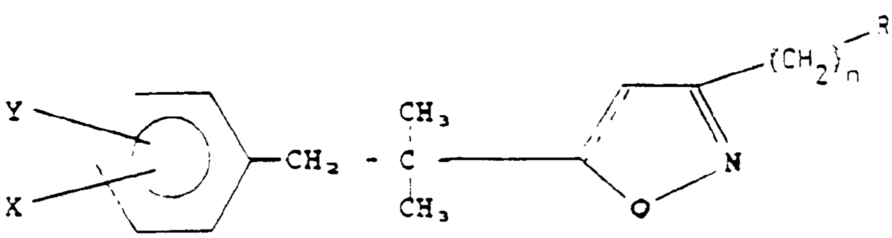 III

wherein X, Y, R and n are as defined above, to a compound of the general formula I wherein Z, X, Y, R and n are as defined above, and $R^1$ and $R^2$ are hydrogen, by a suitable reductive process, or

C) converting an unsaturated ketone of the formula IV

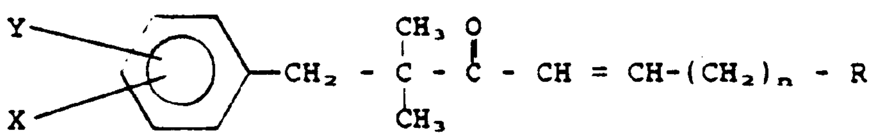 IV

wherein X, Y, R and n are as defined above, to a compound of the general formula I wherein Z is CO, X, Y, R, $R^1$, $R^2$ and n are as defined above, by addition of $HNR^1R^2$, or

D) converting the corresponding derivatives of the formula V

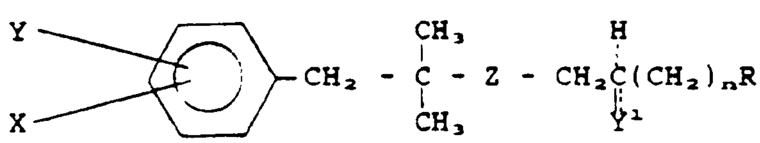 V

wherein X, Y, Z, n and R are as defined above and $Y^1$ represents a group which can be transformed to a $NR^1R^2$ group, by a suitable hydrolytic, reductive, electrochemical, alkylating or other known process, or

E) converting the corresponding compound of the general formula VI

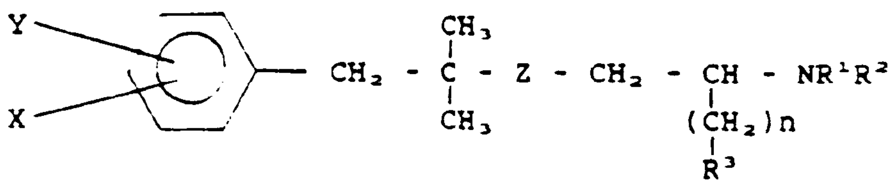 VI

wherein X, Y, Z, n, $R^1$ and $R^2$ are as defined above and $R^3$ represents a group which can be transformed to an alkyl group R defined as above by a suitable reductive, hydrolytic or other known process, or

F) reducing the corresponding compound of the general Formula VII

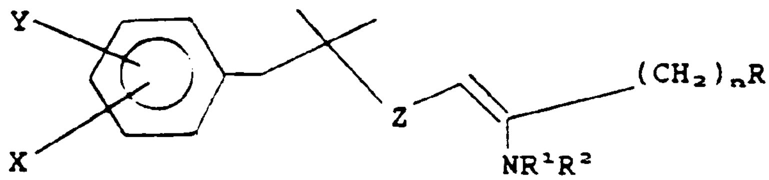 VII

wherein X, Y, Z, R, $R^1$, $R^2$ and n are as defined above, by known processes, or

G) reacting a ketone of the formula VIII

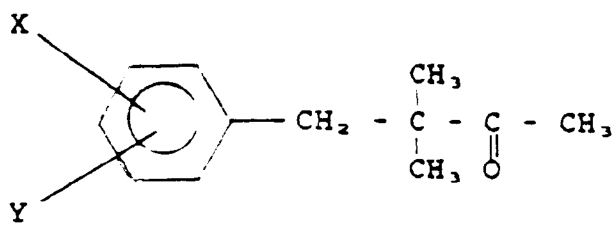 VIII

wherein X and Y are as defined above with a suitable aldehyde $CHO(CH_2)_nR$ and $NHR^1R^2$ wherein R, R′, $R^2$ and n are as defined above, or

H) resolution of a racemate of the formula I, or

J) by converting the corresponding derivatives of the general formula IX

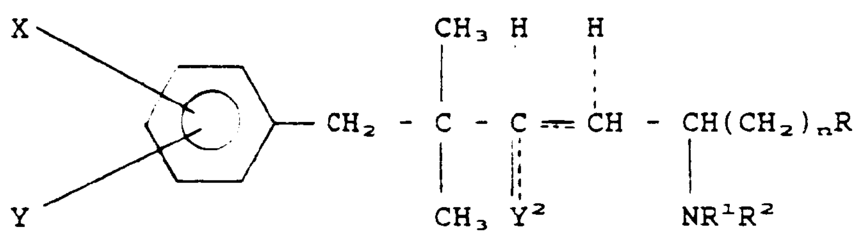 IX

wherein X, Y, n, R, $R^1$ and $R^2$ are as defined above and $CHY^2$ represents a group which can be transformed to Z defined as above by a suitable hydrolytic, oxidative, reductive or other known process, e.g. by oxidation of alcohol to ketone, by reduction of ketone to alcohol, or by cleavage of suitable alcohol or ketone protecting groups, or

K) by converting a compound of the general formula X

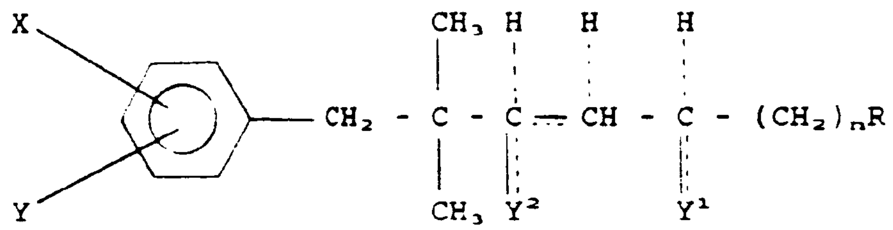 X

to a compound of the general formula I by the combination of the methods D and J, wherein X, Y, R, n, $Y^1$ and $Y^2$ are as defined above and wherein $Y^1$ and $Y^2$ can also be included in a ring.

According to process A) the reducing agent is preferably sodium cyanoborohydride, sodium borohydride, or hydrogen in the presence of a catalyst e.g. Pd/C, Pt or Raney Ni. It is especially preferred to perform the reaction in methanol with sodium cyanoborohydride as reducing agent.

According to process B) the reducing agent is an appropriate reagent preferably chosen from the following: Sodium in liquid ammonia or lithium in an amine solution in the presence of an alcohol, or hydrogen in the presence of a catalyst,or lithium aluminium hydride or sodium borohydride in the presence of a Lewis acid. Most preferred is sodium in liquid ammonia in the presence of t-butanol for the preparation of compound I when Z is CO, and lithium aluminium hydride for the preparation of compound I when Z is CH(OH).

According to process D),the group $Y^1$ in the compound V may be chosen from easily cleaved, chiral or non-chiral amides, carbamates, imines, benzylic amines or other suitable amine protecting groups. Such groups can be trifluoroacetamide, formamide, t-butylcarbamate, N-benzylamine, (S) or (R)-phenethylamine, or chiral Schiff bases such as (+) or (-)-2-hydroxypinanyl-3-ideneamines. In addition $Y^1$ can be groups such as nitro, azido, oxime, hydrazone or imine, which can be transformed to $NH_2$ by known reductive processes. Alternatively $Y^1$ can be $NR^1R^2$ when $R^1$ and/or $R^2$ is hydrogen, which can be alkylated by known processes in a stepwise or direct manner.

The compounds of the formula II can be isolated as such and subsequently converted to a compound of the formula I or the compounds of the formula II can be generated in situ and converted to a compound of the formula I without isolation.

According to process E the group R may be an unsaturated alkyl or a suitable protected alkyne.

When the compound of the formula I are enantiomerically enriched, this can be accomplished by a synthesis directly giving an optically active compound I or a suitable intermediate, or by resolution of racemic I or a suitable intermediate by known processes including chromatographic methods with or without derivatization or by fractional crystallization.

When the compounds of formula I are diastereomerically enriched this can be accomplished by direct synthesis or by chromatographic or crystallographic separations of the compounds or suitable derivatives or intermediates.

## Pharmaceutical preparations

According to the present invention the compounds of the formula I will normally be administered orally, rectally or by injection, in the form of pharmaceutical preparations comprising the active ingredient either as a free base or a pharmaceutically acceptable non-toxic, acid addition salt, e.g. the hydrochloride, hydrobromide, lactate, acetate, phosphate, sulphonate, sulphamate, citrate, tartrate, oxalate and the like in association with a pharmaceutically acceptable dosage form. The dosage form may be a solid, semisolid or liquid preparation. Usually the active substance will constitute between 0.1 and 99% by weight of the preparation, more specifically between 0.5 and 20% by weight for preparations intended for injection and between 0.2 and 50% by weight for preparations suitable for oral administration.

To produce pharmaceutical preparations containing a compound of the formula I in the form of dosage units for oral application the selected sompound may be mixed with a solid excipient, e.g. lactose, saccharose, sorbitol, mannitol, starches such as potato starch, corn starch or amylopectin, cellulose derivatives, a binder such as gelatine or polyninylpyrrolidone, and a lubricant such as magnesium stearate, calcium stearate, polyethylene glycol, waxes, paraffin and the like, and then compressed into tablets. If coated tablets are required, the cores, prepared as described above, may be coated with a concentrated sugar solution which may contain, e.g. gum & arabic, gelatine, talcum, titanium dioxide, and the like. Alternatively, the tablet can be coated with a polymer known to the man in the art, dissolved in a readily volatile organic solvent or mixture of organic solvents or in water. Dyestuffs may be added to these coatings in order to readily distinguish between tablets containing different active substances or different amounts of the active compounds.

For the preparation of soft gelatine capsules, the active substance may be admixed with e.g. a vegetable oil or polyethylene glycol. Hard gelatine capsules may contain granules of the active substance using either the above mentioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Dosage units for rectal application can be solutions or suspensions or can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example, solutions containing from about 0.2 % to about 20 % by weight of the active substance herein described, the balance being sugar and mixture of ethanol, water, glycerol, and propylene glycol. Optionally such liquid preparations may contain colouring agents, flavouring agents, saccarine and carboxymethylcellulose as a thickening agent or other excipients known to the man in the art.

Solutions for parenteral applications can be prepared in an aqueous solution of a water-soluble pharmaceutically acceptable salt of the active substance preferably in a concentration of from about 0.5 % to about 10 % by weight. These solutions may also contain stabilizing agents and/or buffering agents and may conveniently be provided in various dosage unit ampoules.

Suitable daily doses of the compounds of the invention in therapeutical treatment of humans are 100 to 500 mg at peroral administration and 20 to 100 mg at parenteral administration.

## EXAMPLES

### Example 1 (Method A)

#### 5-amino-2,2-dimethyl-1-(4-fluorophenyl)-3-heptanone oxalate

To a solution of 2,2-dimetyl-1-(4-fluorophenyl)3,5-heptanedione (3.02 g, 12 mmol), dry ammonium acetate (7.4 g, 96 mmol) and sodium cyanoborohydride (1.13 g, 18 mmol) and molecular sieves in dry methanol (150 ml) a few milliliters of acetic acid were added to adjust the pH to 6.5. After stirring for 30 h at room temperatue the reaction was interrupted by adding acid to pH 2. After filtration and evaporation of the solvent the residue was dissolved in water, washed with ether and made alkaline. After extraction with ether, drying and removal of the solvent a crude oil (0.33 g) was obtained, which was precipitated as an oxalate and recrystallized from 2-propanol to give 0.15 g of the title product.
M.p. 125-127 °C.

### Example 2 (Method B)

#### 5-Amino-2,2-dimethyl-1-(4-fluorophenyl)-3-heptanone

To a solution of 3-ethyl-5-[1-(4-fluorophenyl)-2-methyl-2-propyl]-isoxazole (0.4 g, 1.6 mmol) and 2-methyl-2-propanol (0.5 g, 6.7 mmol) in 5 ml THF and 30 ml ammonia at -40 °C sodium (0.2 g, 9mmol) in small pieces was added during 15 min. Ammoniumechloride (0.5 g) was added and ammonia was evaporated. The residue was taken up in either (10 ml) and washed with water. The organic phase was dried and the solvent evaporated to yield 0.15 g of the title product as an oil.

### Example 3 (Method C)

#### 5-amino-2,2-dimethyl-1-(4-fluorophenyl)-3-heptanone oxalate

2,2-Dimethyl-1-(4-fluorophenyl-hept-4-en-3-one (0.22 g, 1 mmol) was added to an excess of $NH_3$ (g) in 10 ml ethanol and stirred at ambient temperature for 24 h. The solvent was evaporated and the residue was taken up in $H_2O$ and diethyl ether. The pH was adjusted to 1.5 with 2N HCL, and the phases were separated. The aqueous phase was made alkaline (pH9) and extracted with ether. The organic phase was dried ($Na_2SO_4$) and the solvent evaporated. The residue was dissolved in 3 ml of diisopropyl ether and the title compound was precipitated with oxalic acid (0.08 g, 0.6 mmol). Yield 0.05 g.
M.p. 125-127 °C (after recrystallization).

Example 4 (Method D)

5-Amino-2,2-dimethyl-1-(4-fluorophenyl)-3-heptanone

A solution of 5-[(trifluoroacetyl)amino]-2,2-dimethyl-1-(4-fluorophenyl)-3-heptanone (50 mg, 0.14 mmol) and barium hydroxide octahydrate (135 mg, 0.43 mmol) in methanol/water (2/1, 3 ml), was stirred at ambient temperature for 4 h. Water was added and the mixture was extracted with ether. The organic phase was dried and evaporated to yield 28 mg of the title product.

Examples 5 and 6 (Method H)

The racemate of 5-amino-2,2-dimethyl-1-(4-fluorophenyl)-3-heptanone was separated into its enantiomers by fractional recrystallization of the (+) and (-) tartrates from ethanol. The enantiomeric purity was determined by gas cromatography (GC) on the (S)-O-methylmandelic amide.
The (+) enantiomer >96% e.e. (Example 5) had the following data:
M.p. ((-)-tartrate) 122-122.5 °C. $[\alpha]_d^{25}$ = +52°
(base,c=2.7,$CHCl_3$)
The (-) enantiomer >96% e.e. (Example 6) had the following data:
M.p. ((+)-tartrate) 120-121.5 °C. $[\alpha]_d^{25}$ = -42°
(base,c=3.1,$CHCl_3$)

Example 7 (Method A)

5-Amino-2,2-dimethyl-1-(4-fluorophenyl)-3-heptanol oxalate

To a solution of 6,6-dimethyl-7-(4-fluorophenyl)-5-hydroxy-3-heptanone (37.0 g, 150 mmol) in 300 ml methanol, ammonium acetate (64 g, 800 mmol), sodium cyanoborohydride (10.0 g, 150 mmol) and molecular sieves (size 3 A, 1.0 g) were added. The mixture was stirred at ambient temperature for 2 h, filtered and the solvent was evaporated. The residue was taken up in diethyl ether and 2 M HCl. The pH was adjusted to 9 by the addition of 2 M NaOH and the phases were separated. The organic phase was dried and evaporated. The residue was dissolved in 250 ml diisopropyl ether and oxalic acid dihydrate (18.9 g, 150 mmol), dissolved in 60 ml ethanol, was added to precipitate 16.3 g of the title product. M.p. 152-154 °C.

In an analogous way the following compounds were prepared:

Example 8

5-Amino-1-(4-fluorophenyl)-2,2,6-trimethyl-3-heptanol oxalate
M.p. 158-160 °C.

Example 9

5-Amino-2,2-dimethyl-1-(4-fluorophenyl)-3-decanol oxalate
M.p. 130-132 °C.

Example 10 (Method D)

5-Amino-2,2-dimethyl-1-(4-fluorophenyl)-3-heptanol oxalate

To a suspension of lithium aluminiumhydride (0.28 g, 7.5 mmol) in 50 ml THF a solution of 6,6-dimethyl-7-(4-fluorophenyl)-5-hydroxy-3-heptanone oxime (0.79 g, 2.9 mmol) in 5 ml THF was added over a period of 5 minutes at room temperature. The mixture was heated to reflux for 2 h and then cooled to 0°C. Diethyl ether (20 ml) and saturated sodium sulfate solution (10 ml) were slowly added and the white precipitate was filtered off. The filtrate was dried and the solvent was evaporated. The residue was dissolved in diisopropylether (3 ml) and a solution of oxalic acid dihydrate (0.30 g, 2.4 mmol) in ethanol (1 ml) was added to yield 0.34 g of the title compound. M.p. 152-154 °C.

Example 11 (Method J)

5-Amino-2,2-dimethyl-1-(4-fluorophenyl)-3-heptanone oxalate

To a suspension of chromium(VI)oxide (9.0 g, 90 mmol) in 150 ml pyridine a solution of 5-amino-2,2-dimethyl-1-(4-fluorophenyl)-3-heptanol oxalate (10.3 g, 30 mmol) in 100 ml of pyridine was added at room temperature over a period of 15 min. The mixture was stirred for 2.5 h and then poured into 600 ml of $H_2O$ and 500 ml of diethyl ether. The pH was adjusted to 9.5, and after separation the ether phase was treated with carbon, dried and evaporated. The residue was dissolved in 50 ml diisopropyl ether and a solution of oxalic acid dihydrate (2.2 g, 17 mmol) in 5 ml ethanol was added. The precipitate was collected and recrystallized from 45 ml diisopropyl ether/ethanol (2:1), to yield 2.0 g of the title compound. M.p. 126-127 °C.

In an analogous way the following compound was prepared:

Example 12

5-Amino-1-(4-fluorophenyl)-2,2,6-trimethyl-3-heptanone oxalate.
M.p. 148-149 °C.

The compounds according to the invention are characterized by standard spectroscopic techniques, e.g. $^1H$ NMR and $^{13}C$ NMR. Data are given in the following Tables 1 and 2. All data except for Example 9 are related to the respective base. For Example 9 the salt has been used.

Table 1 $^1H$ NMR

| Compound according to example No | Solvent | Chemical shift δ (ppm) |
|---|---|---|
| 1,2,3,4,5,6,11 | $CDCl_3$ | 7.1 (m, 2H), 6.9 (t, 2H), 3.1 (m, 1H), 2.8 (AB, 2H), 2.4 (AB, 2H), 1.3 (m, 2H), 1.11 (s, 6H), 0.9 (t, 3H) |
| 12 | $CDCl_3$ | 7.1 (m, 2H), 6.9 (t, 2H), 3.05 (m, 1H), 2.8 (AB, 2H), 2.5 (AB, 2H), 1.6 (m, 1H), 1.5 (broad s, 2H), 1.13 (s, 6H), 0.90 (d, 3H), 0.88 (d, 3H). |

Table 2 $^{13}C$ NMR

| Compound according to example No | Solvent | Chemical shift δ (ppm) |
|---|---|---|
| 7, 10 | $CDCl_3$ | 161.5(d), 135.1(d), 132.5(d), 114.3(d), 78.6, 74.3, 54.9, 51.4, 43.7, 43.65, 38.4, 35.9, 34.7, 34.0, 28.8, 23.3, 23.25, 22.3, 22.25, 11.0, 10.1 |
| 8 | $CDCl_3$ | 161.5(d), 135.1(d), 132.3(d), 114.3(d), 114.3(d), 78.8, 74.5, 58.6, 55.4, 43.8, 43.7, 38.6, 38.4, 35.5, 33.5, 33.3, 32.5, 23.3, 23.2, 22.3, 22.1, 19.5, 19.0, 18.9, 17.0. |
| 9 | $CD_3OD$ | 163.1(d), 136.1(d), 133.5(d), 115.5(d), 78.5, 75.1, 53.5, 51.5, 44.2, 40.0, 34.5, 34.1, 32.9, 26.4, 26.0, 23.64, 23.59, 23.5, 23.0, 22.8, 14.5. |

Example 13 (Method D)

(+)- and (-)-5-Amino-2,2-dimethyl-1-(4-fluorophenyl)-3-heptanone

To a solution of (S)-1-phenylethylamine (5,6 g, 43 mmol) in ethanol (50 ml) was added 2,2-dimethyl-1-(fluorophenyl)hept-4-en-3-one (10 g, 43 mmol) at 0 °C. The mixture was left with stirring at room temperature over night. Evaporation of the solvent left a mixture of reaction products(15 g) which were separated on $SiO_2$ / $CHCl_3$: methanol: konc $NH_3$ (750: 3: 0.5). Two fractions were collected and gave the two (S)-1-phenylethylamine adducts (A: 3.2 g and B: 3.9 g).

A: 7.4 (s, 5H), 7.2-6.8 (m, 4H), 3.65(m, 1H), 2.9 (m, 1H), 2.7 (s, 2H), 2.4 (dd, 2H), 1.9 (broad s, 1H), 1.7-1.2 (m, 2H), 1.3 (d, 3H), 1.05 (s, 6H), 0.8 (t, 3H)

B: 7.4 (s, 5H), 7.3-6.8 (m, 4H), 3.65 (m, 1H), 2.9 (m, 1H), 2.8 (s, 2H), 2.55 (dd, 2H), 1.8-1.2 (m, 2H), 1.6 (broad s, 1H), 1.35 (d, 3H), 1.15 (s,6H), 0.8 (t,3H)

A mixture of enantiomeric pure (5-(N-(1-(S)-methylbenzyl]-amino)-2,2-dimethyl-1-(4-fluorophenyl)-3-heptanone (fraction B: 362 mg, 1 mmol) in methanol-$H_2O$-HOAc (3.5 ml + 3 ml + 60 μl) and 10% $Pd(OH)_2/C$ (120 mg) was stirred in an atmosphere of $H_2$ at room temperature over night. After filtration, the reaction mixture was concentrated in vacuum and the residue partitioned between diethylether and aq.$NaHCO_3$. The etheral

phase was dried ($Na_2SO_4$) and evaporated to dryness to give an oil (159 mg) of 5-amino-2,2-dimethyl-1-(4-fluorophenyl)-3-heptanone. The enantiomeric purity of the product was more than 95% as determined by GC on its (S)-O-methylmandelic amide. The formed (+)-5-amino-2,2-dimethyl-1-(4-fluorophenyl)-3-heptanone was shown to be identical to the compound in example 5. In an analogous way the fraction A was converted to the (-)-isomer.

Example 14 (Method J )

By the procedure described in Example 11 the compound 5-amino-2,2-dimethyl-1-(3-trifluoromethyl-phenyl)-3-heptanone oxalate was prepared. M.p. 98-101 °C.

Example 15 (Method A)

By the procedure described in Example 7 the compound 5-amino-1-(4-tolyl)-2,2,7-trimethyl-3-octanol oxalate was prepared. M.p. 173-176 °C.

Example 16 (Method J )

By the procedure described in Example 11 the compound 5-amino-1-(4-tolyl)-2,2,7-trimethyl-3-octanone oxalate was prepared. M.p. 148-150 °C.

Example 17 (Method C)

By the procedure described in Example 3 the compound 5-amino-1-(4-bromophenyl)-2,2-dimethyl-3-hexanone oxalate was prepared. M.p. 104-108 °C.

Example 18

By the procedure described in Example 3 the compound 5-amino-1-(2,4-dichlorophenyl)-2,2-dimethyl-3-octanone oxalate was prepared. M.p. 98-101 °C.

Example 19

By the procedure described in Example 3 the compound 5-amino-2,2-dimethyl-1-phenyl-3-heptanone oxalate was prepared. M.p. 142-144 °C.

Example 20

By the procedure described in Example 3 the compound 5-amino-1-phenyl-2,2,7-trimethyl-3-octanone oxalate was prepared. M.p. 147-149 °C.

Example 21

By the procedure described in Example 3 the compound 5-amino-1-(3,5-dichlorophenyl)-2,2-dimethyl-3-hexanone oxalate was prepared. M.p. 182-184 °C.

Example 22

By the procedure described in Example 3 the compound 5-amino-2,2-dimethyl-1-(4-fluorophenyl)-6-phenyl-3-hexanone oxalate was prepared. M.p. 165-168 °C.

Example 23

By the procedure described in Example 3 the compound 5-amino-1,5-bis(4-fluorophenyl)-2,2-dimethyl-3-pentanone oxalate was prepared. M.p. 157-161 °C.

Example 24

By the procedure described in Example 3 the compound 5-amino-5-cyclohexyl-2,2-dimethyl-1-(4-fluorophenyl)-3-pentanone oxalate was prepared. M.p. 150-152 °C.

Example 25

By the procedure described in Example 3 the compound 5-amino-1-(4-cyanophenyl)-2,2-dimethyl-3-heptanone oxalate was prepared. M.p. 152-155 °C.

Example 26

By the procedure described in Example 3 the compound 5-amino-2,2-dimethyl-1-(4-fluorophenyl)-3-pentanone oxalate was prepared. M.p. 122-125 °C.

Example 27 (Method G)

2,2-Dimethyl-5-dimethylamino-1-(4-fluorophenyl)-3-pentanone oxalate

A mixture of 3,3-dimethyl-4-(4-fluorophenyl)-2-butanone (1.94 g, 10 mmol), dimethylamine hydrochloride (1.06 g, 13 mmol), paraformaldehyde (0.40 g, 4.4 mmol) and 0.12 ml 2 N HCl in 2 ml ethanol was refluxed for 16 h and poured into 20 ml of hot acetone. The precipitate was filtered off and recristallized from aceton/ethanol (10:1) to yield 1.3 g of the title compound. M.p. 152-153°C.

Example 28 (Method J)

5- Amino-1-(3,5-dichlorophenyl)-2,2-dimethyl-3-hexanol oxalate

To a solution of 5-amino-1-(3,5-dichlorophenyl)-2,2-dimethyl-3-hexanone (1.01 g, 3.5 mmol) in 20 ml ethanol was added sodium borohydride (132 mg, 3.5 mmol) at 5 °C. The mixture was stirred for 30 min at 5 °C, poured into a mixture of dilute cold hydrochloric acid and ether. The aqueous phase was made alkaline and extracted with ether. The ether phase was dried and the solvent evaporated. The residue was dissolved in 15 ml diisopropyl ether and oxalic acid dihydrate (353 mg, 2.8 mmol), dissolved in 1 ml ethanol was added to precipitate 720 mg of the title compound.
M.p. 170-173 °C.

Example 29

By the procedure described in Example 3 the compound 2,2-Dimethyl-1-(4-fluorophenyl)-5-(N-methylamino)-3-heptanone oxalate was prepared. M.p. 118-120°C.

Example 30

By the procedure described in Example 3 the compound 2,2-Dimethyl-5-(N,N-dimethylamino)-1-(4-fluorophenyl)-3-heptanone oxalate was prepared. M.p. 147-149°C.

Example 31 (Method E)

5-Amino-2,2-dimethyl-1-(4-fluorophenyl)-hept-6-yn-3-one

To a solution of 5-amino-2,2-dimethyl-1-(4-fluorophenyl)-7-trimethylsilylhept-6-yn-3-one (64.5 mg, 0.2 mmol) in THF (1 ml) was added an excess of tetrabutylammonium fluoride trihydrate and the reaction mixture was left at room temperature over night. Workup gave 37 mg of the title compound as an oil. $^1H$ NMR; ($CDCl_3$) $\delta$ 6.90-7.10(m, 4H), 4.08(dt, J=8Hz and 2Hz, 1H), 2.81-2.70 (m, 4H), 2.28(d, J=2Hz, 1H), 1.75(broad s, 2H), 1.12(s, 6H). The oil was dissolved in ethanol and treated with a solution of oxalic acid (18.5 mg, 0.15 mmol) in the same solvent. The precipitate was isolated to furnish 2.2 mg of 5-amino-2,2-dimethyl-1-(4-fluorophenyl)-6-heptyn-3-one oxalate.
M.p. 120-126°C.

Example 32 (Method D)

2,2-Dimethyl-1-(4-fluorophenyl)-5-N-(methylamino)-3-heptanone

To a solution of 5-amino-2,2-dimethyl-1-(4-fluorophenyl)-3-heptanone (140 mg, 0.56 mmol) in 3 ml ethanol was added methyl iodide (79 mg, 0.56 mmol) and triethylamine (56 mg, 0.56 mmol) at ambient temperature. The mixture was stirred at 40°C for 2h and the solvent removed. The residue was taken up in ether and washed with water. The organic phase was dried and the solvent evaporated to yield 95 mg of the title product as an oil, which showed the expected spectroscopic characteristics.

Pharmaceutical preparations

The following examples illustrate the preparation of pharmaceutical compositions to be used in the method of the invention. For the preparation of tablets the following compositions can be made.

Example 33

| | |
|---|---|
| Compound according to Example 7 | 50 g |
| Lactose | 85 g |
| Potato starch | 40 g |
| Polyvinylpyrrolidone | 5 g |
| Microcrystalline cellulose | 18 g |
| Magnesium stearate | 2 g |

Example 34

| | |
|---|---|
| Compound according to Example 1 | 100 g |
| Lactose | 90 g |
| Potato starch | 50 g |
| Polyvinylpyrrolidone | 5 g |
| Microcrystalline cellulose | 23 g |
| Magnesium stearate | 2 g |

From the above compositions 1 000 tablets can be made, containing 50 mg and 100 mg of active substance, respectively. If desired, the obtained tablets can be film coated with e.g. methyl cellulose in an organic solvent or using water.

Example 35

Example of solution for intravenous or intramuscular injection.

| | |
|---|---|
| Compound according to Example 1 | 60 g |
| Water for injection | ad 1000 ml |

The active compound shall be dissolved in water to a final volume of 1000 ml and the solution filtered through a sterile 0.22 μm filter and aseptically dispensed into 1 ml sterile ampoules, which then are sealed.

PHARMACOLOGICAL METHOD

Potentiation of cholinergic responses

Male rats (Sprague Dawley Alab Laboratorietjänst AB, Sollentuna, Sweden) weighing 150-180 g, 35-40 days of age, were used. The animals were allowed free access to food (R3, Ewos AB, Södertälje, Sweden) and water until the start of the experiments. The test compounds were injected intraperitoneally (i.p.) 30 minutes before injection of the muscarine agonist oxotremorine (OTM) into the neck (s.c.). Tremor intensity was assessed visually for a 60 minutes period following the injection of OTMN in rat housed in Macrolon cages (25x25x30 cm) (three per cage). The threshold dose of OTMN for eliciting tremor in rat is about 200 µg/kg.

Tremor intensity was scored using the following rating scale: 0 = no tremor; 1 = slight tremor (moderate, discontinuous tremor; 2 = strong tremor (intense, continuous tremor involving the whole body). The animals were scored 5, 10, 15, 30 and 60 minutes after injection of OTMN. The tremor was scored for a period of 30 seconds.

The values for each observation were summed over the entire observation period (5-60 minutes) and their median values for each group were calculated (total median score). Mann-Whitney U-test (two-tailed) was used to determine differences between the control OTMN group and test groups receiving the compounds + OTMN. It should be noted concurrently with each compound tested.

The lowest dose causing a significant ($p<0.05$) potentiation of the OTMN response was determined from log-dose response curves.

In the following Table 3 data are shown on the testing for the potentiation of oxotremorine induced tremor in rat of the known compound alaproclate with the formula

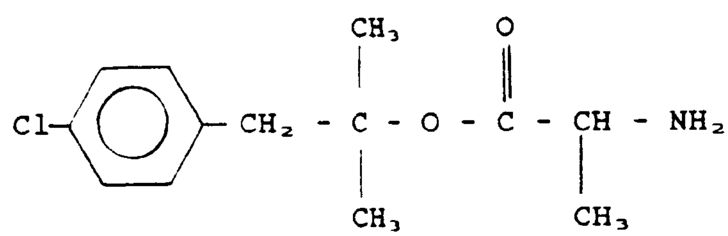

and compounds of the formula

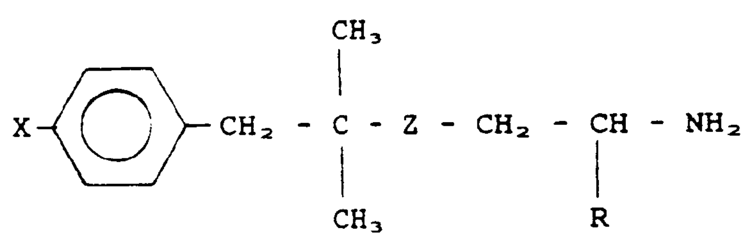

Table 3

| Compound according to example no or ref. | X | Z | R | Potentiation of OTMN-induced tremor in rat mg/kg |
|---|---|---|---|---|
| Alaproclate | | | | 5.0 |
| 1,2,3,4,11 | F | CO | $C_2H_5$ | 0.63 |
| 5 | (-) F | CO | $C_2H_5$ | 0.63 |
| 6 | (+) F | CO | $C_2H_5$ | 0.63 |
| 12 | F | CO | $CH(CH_3)_2$ | 2.5 |
| 7,10 | F | CH(OH) | $C_2H_5$ | 2.5 |
| 8 | F | CH(OH) | $CH(CH_3)_2$ | 10.0 |
| 9 | F | CH(OH) | $(CH_2)_4CH_3$ | 5.0 |

The compounds of the general formula I potently potentiate the tremor inducing effect of oxotremorine, which indicates a facilitation of central cholinergic responses.

## Claims

**Claims for the following Contracting States : AT, BE, CH, DE, FR, GB, IT, LI,LU, NL, SE**

1. A compound of the general formula I

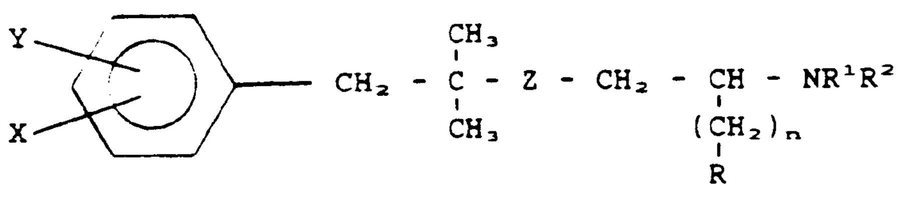

I

wherein n is 0 or 1, Z is CO or CH(OH), $R^1$ and $R^2$ are equal or different and, when equal, hydrogen, methyl, ethyl or propyl and, when different, $R^1$ is hydrogen and $R^2$ is methyl, ethyl, propyl or isopropyl, X and Y represent hydrogen, halogen, $CF_3$, CN or an alkyl group, straight or branched having 1-3 carbon atoms, in the ortho, meta or para position provided that when X is in the ortho position and other than hydrogen then Y is other than hydrogen, and when Y is in the ortho position and other than hydrogen then X is other than hydrogen, R represents a straight or branched saturated or unsaturated 1-6 carbon alkyl group, a 3-6 carbon cycloalkyl group, a phenyl group, which may be unsubstituted or may carry a ring substituent $X^1$, $X^1$ preferably being a halogen, or R represents hydrogen, in racemic or optically active form and when Z is CH(OH) also mixtures of diastereomers or a pure diastereomer, with the proviso that the racemate of the compound wherein $R^1$ and $R^2$ is hydrogen, X is p-Cl, Y is hydrogen, Z is CO, n is 0 and R is methyl is excluded; or a pharmaceutically acceptable salt thereof.

2. A compound according to claim 1, wherein $R^1$ and/or $R^2$ is hydrogen or methyl.

3. A compound according to claim 1, wherein $R^1$ and $R^2$ is hydrogen.

4. A compound according to claim 1, wherein R, when alkyl, is saturated straight or branched having 1-3 carbon atoms in the chain.

5. A compound according to claim 1, wherein R, when cycloalkyl, has 5-6 carbon atoms in the ring.

6. A compound according to claim 1, wherein X, Y and $X^1$ are in the meta and/or para positions.

7. A compound according to claim 1, wherein halogen X, Y and $X^1$ is bromo, chloro or fluoro in the meta and/or para position.

8. A compound according to claim 7, wherein halogen X, Y and $X^1$ is para fluoro.

9. A compound according to claim 1, wherein X and/or Y is $CF_3$ in the meta position when being the monosubstituent.

10. A compound according to claim 1, wherein X and/or Y is CN in the para position when being the monosubstituent.

11. A compound according to claim 1, wherein X and/or Y is methyl in the para position when being the monosubstituent.

12. A compound according to claim 9, 10 or 11, wherein R is a saturated alkyl group straight or branched having 1-3 carbon atoms in the chain.

13. A compound according to claim 1, wherein n is 0, Z is as defined above, $R^1$ and $R^2$ are both hydrogen, X is para-C1 or para-F and R is an ethyl or an isopropyl group.

14. A compound according to claim 13 wherein Z is CO, X is F and R is ethyl.

15. A compound according to claim 13, wherein Z is CO, X is F and R is isopropyl.

16. A compound according to claim 13, wherein Z is CH(OH), X is F and R is ethyl.

17. A compound of the general formula Ia

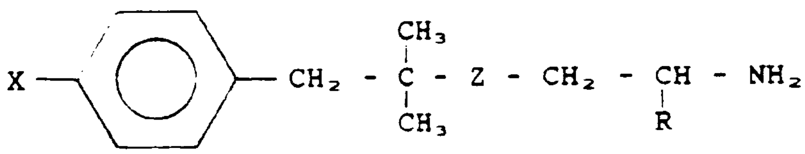

Ia

or a pharmaceutically acceptable salt thereof, wherein Z is CO or CH(OH), X is halogen and R is a straight or branched alkyl group having 1-5 carbon atoms in the chain in racemic or optically active form and when Z is CH(OH) also mixtures of diastereomers or the pure diastereomers, with the proviso that the racemate of the compound, wherein X is Cl, Z is CO and R is methyl is excluded.

18. A compound according to claim 17, wherein Z is as defined above, X is Cl or F and R is an ethyl or an isopropyl group.

19. A compound according to claim 17 wherein Z is CO, X is F and R is ethyl.

20. A compound according to claim 17, wherein Z is CO, X is F and R is isopropyl.

21. A compound according to claim 17, wherein Z is CH(OH), X is F and R is ethyl.

22. A compound according to one or more of the preceding claims, which is the (+)enantiomer.

23. A compound according to one or more of the preceding claims, which is the (-)enantiomer.

24. A compound according to claim 1 exhibiting potentiating effect on central cholinergic responses.

25. A compound according to claim 1 effective on mental disturbances.

26. A process for the preparation of a compound with the general formula I

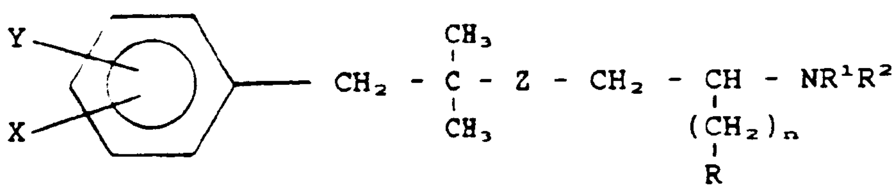

I

wherein Z, X, Y, R, $R^1$, $R^2$ and are as defined above, by

A) reacting a compound of the formula II

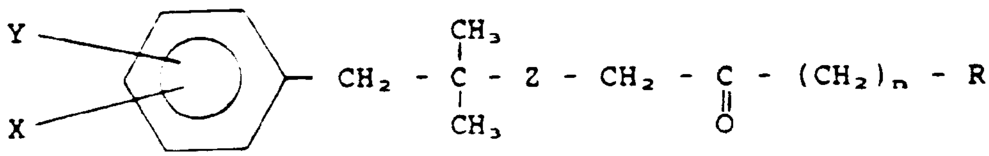

II

wherein Z, X, Y, R and n are as defined above, with ammonia, an ammonium salt or a suitabte amine derivative in the presence of a suitable reducing agent in a stepwise or direct manner, or

B) reduction of the corresponding derivative of the formula III

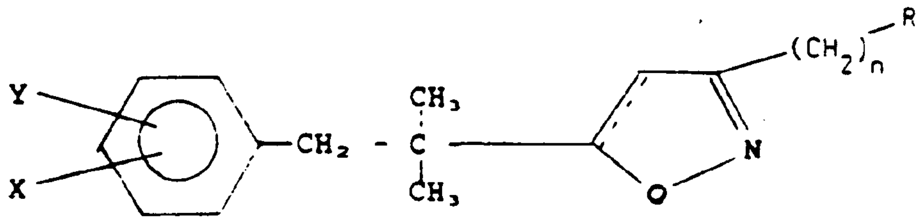

III

wherein X, Y, R and n are as defined above, to a compound of the general formula I wherein Z, X, Y, R and n are as defined above, and $R^1$ and $R^2$ are hydrogen, by a suitable reductive process, or

C) converting an unsaturated ketone of the formula IV

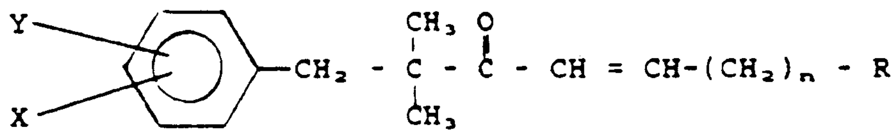

IV

wherein X, Y, R and n are as defined above, to a compound of the generat formula I wherein Z is CO, X, Y, R, $R^1$, $R^2$ and n are as defined above, by addition with $HNR^1R^2$, or

D) converting the corresponding derivatives of the formula V

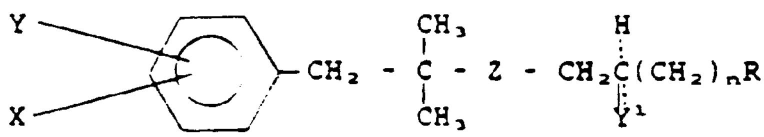

V

wherein X, Y, Z, n and R are as defined above and $Y^1$ represents a group which can be transformed to a $NR^1R^2$ group, by a suitable hydrolytic, reductive, electrochemical, alkylating or other known process, or

E) converting the corresponding compound of the general formula VI

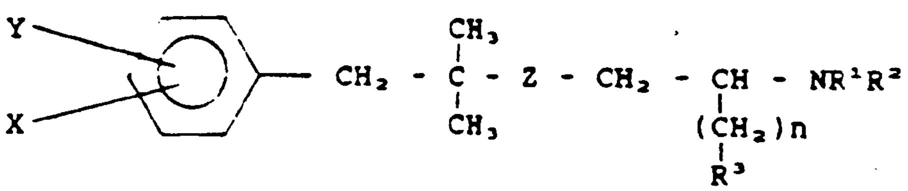

VI

wherein X, Y, Z, n, $R^1$ and $R^2$ are as defined above and $R^3$ represents a group which can be transformed to an alkyl group R defined as above by a suitable reductive, hydrolytic or other known process, or

F) reducing the corresponding compound of the general formula VII

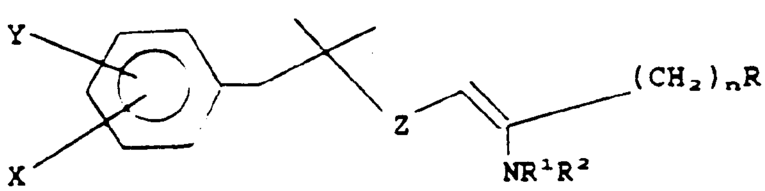

VII

wherein X, Y, Z, R, $R^1$, $R^2$ and n are as defined above, by known processes, or

G) reacting a ketone of the formula VIII

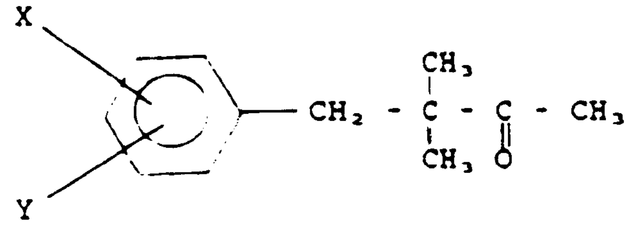

VIII

wherein X and Y are as defined above with a suitable aldehyde $CHO(CH_2)_nR$ and $NHR^1R^2$ wherein R,R',$R^2$ and n are as defined above, or

H) resolution of a racemate of the formula I, or

J) by converting the corresponding derivatives of the general formula IX

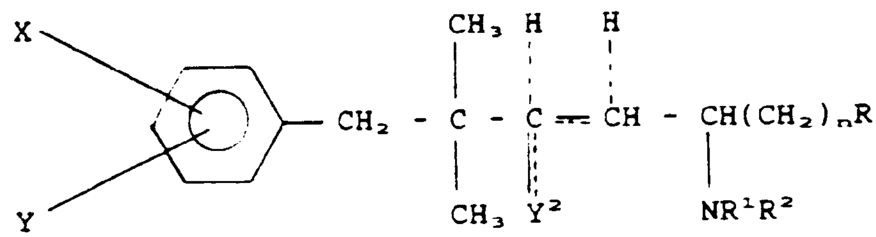

IX

wherein X, Y, n, R, $R^1$ and $R^2$ are as defined above and $CHY^2$ represents a group which can be transformed to Z defined as above by a suitable hydrolytic, oxidative, reductive or other known process, or

K) by converting a compound of the general formula X

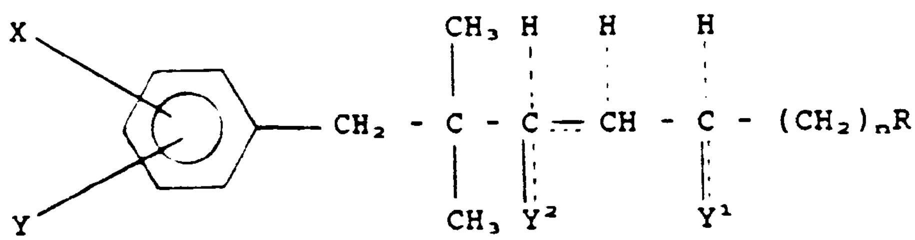

X

to a compound of the general formula I by the combination of the methods D and J, wherein X, Y, R, n, $Y^1$ and $Y^2$ are as defined above and wherein $Y^1$ and $Y^2$ can also be included in a ring, and if desired converting a base obtained into a pharmaceutically acceptable acid addition salt or converting a salt obtained into the base.

27. A pharmaceutical preparation containing as active ingredient a compound according to any of the claims 1-25.

28. Use of a compound with the general formula I according to claim 1 or a pharmaceutically acceptable salt thereof for the preparation of a pharmaceutical formulation with effect on mental disturbances.

29. Use of a compound with the general formula I according to claim 1 or a pharmaceutically acceptable salt thereof for the preparation of a pharmaceutical formulation with effect in obtaining potentiating effect on cholinergic responses in a patient having mental disturbances.

**Claims for the following Contracting States: GR, ES**

1. A process for the preparation of a compound with the general formula I

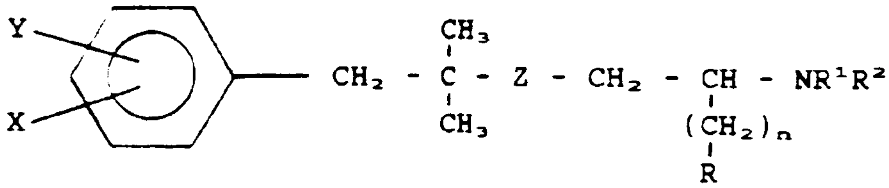

I

wherein n is 0 or 1, Z is CO or CH(OH), $R^1$ and $R^2$ are equal or different and, when equal, hydrogen, methyl, ethyl or propyl and, when different, $R^1$ is hydrogen and $R^2$ is methyl, ethyl, propyl or isopropyl, X and Y represent hydrogen, halogen, $CF_3$, CN or an alkyl group, straight or branched having 1-3 carbon atoms, in the ortho, meta or para position provided that when X is in the ortho position and other than hydrogen then Y is other than hydrogen, and when Y is in the ortho position and other than hydrogen then X is other than hydrogen, R represents a straight or branched saturated or unsaturated 1-6 carbon alkyl group, a 3-6 carbon cycloalkyl group, a phenyl group, which may be unsubstituted or may carry a ring substituent $X^1$, $X^1$ preferably being a halogen, or R represents hydrogen, in racemic or optically active form and when Z is CH(OH) also mixtures of diastereomers or a pure diastereomer, with the proviso that the racemate of the compound wherein $R^1$ and $R^2$ is hydrogen, X is p-Cl, Y is hydrogen, Z is CO, n is 0 and R is methyl is excluded; or a pharmaceutically acceptable salt thereof, by

A) reacting a compound of the formula II

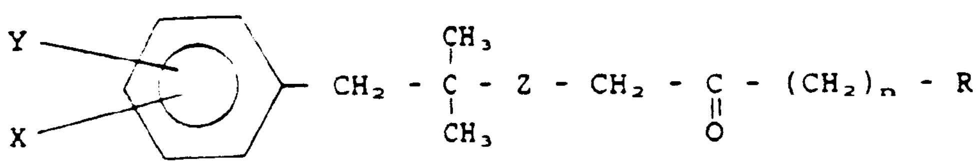

II

wherein Z, X, Y, R and n are as defined above, with ammonia, an ammonium salt or a suitable amine derivative in the presence of a suitable reducing agent in a stepwise or direct manner, or

B) reduction of the corresponding derivative of the formula III

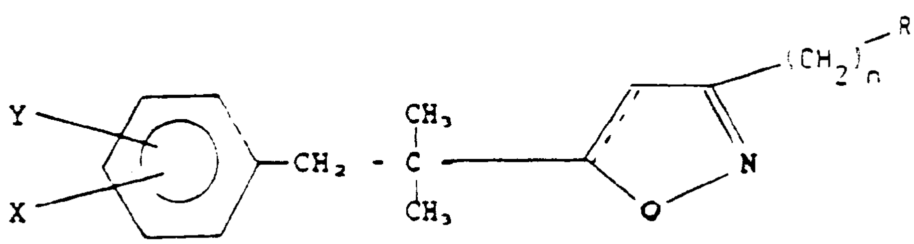

III

wherein X, Y, R and n are as defined above, to a compound of the general formula I wherein Z, X, Y, R and n are as defined above, and $R^1$ and $R^2$ are hydrogen, by a suitable reductive process, or

C) converting an unsaturated ketone of the formula IV

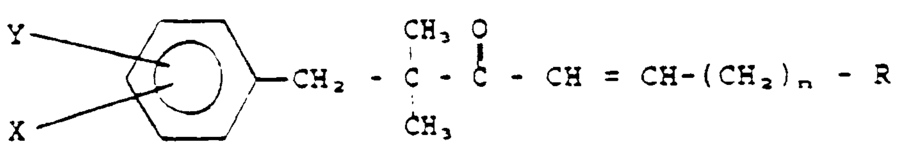

IV

wherein X, Y, R and n are as defined above, to a compound of the general formula I wherein Z is CO, X, Y, R, $R^1$, $R^2$ and n are as defined above, by addition of $HNR^1R^2$, or

D) converting the corresponding derivatives of the formula V

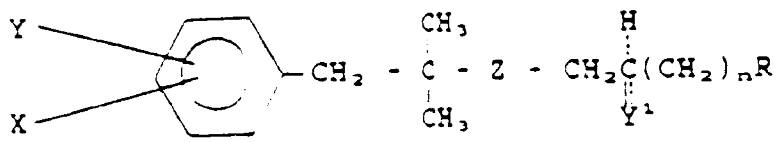

V

wherein X, Y, Z, n and R are as defined above and $Y^1$ represents a group which can be transformed to a $NR^1R^2$ group, by a suitable hydrolytic, reductive, electrochemical, alkylating or other known process, or
E) converting the corresponding compound of the general formula VI

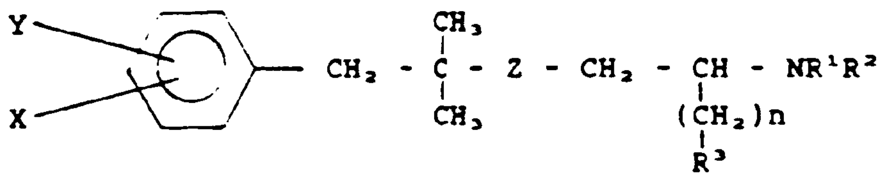

VI

wherein X, Y, Z, n, $R^1$ and $R^2$ are as defined above and $R^3$ represents a group which can be transformed to an alkyl group R defined as above by a suitable reductive, hydrolytic or other known process, or
F) reducing the corresponding compound of the general formula VII

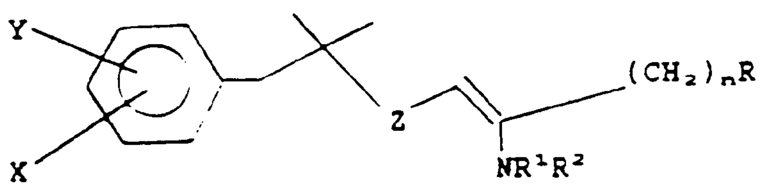

VII

wherein X, Y, Z, R, $R^1$, $R^2$ and n are as defined above, by known processes, or
G) reacting a ketone of the formula VIII

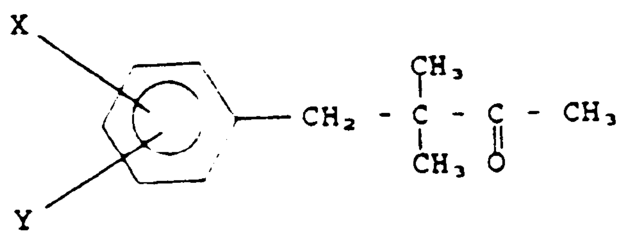

VIII

wherein X and Y are as defined above with a suitable aldehyde $CHO(CH_2)_nR$ and $NHR^1R^2$ wherein R,R′,$R^2$ and n are as defined above, or
H) resolution of a racemate of the formula I, or
J) by converting the corresponding derivatives of the general formula IX

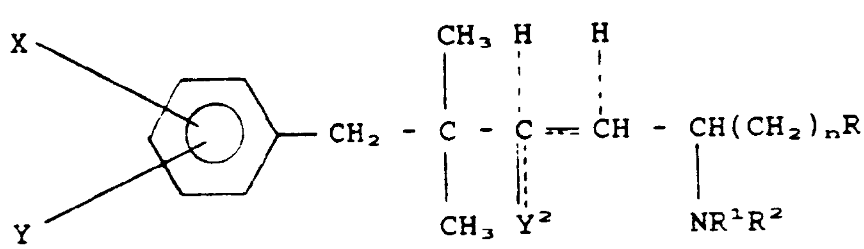

IX

wherein X, Y, n, R, $R^1$ and $R^2$ are as defined above and $CHY^2$ represents a group which can be transformed to Z defined as above by a suitable hydrolytic, oxidative, reductive or other known process, or
K) by converting a compound of the general formula X

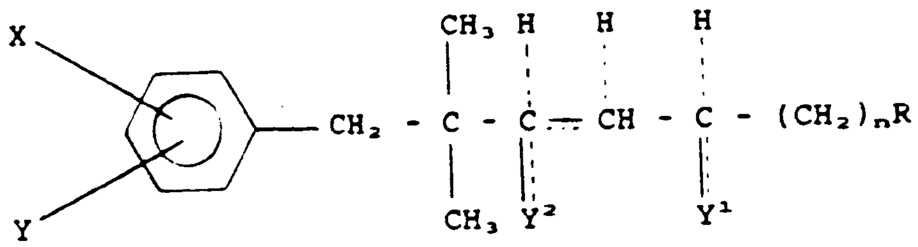

X

to a compound of the general formula I by the combination of the methods D and J, wherein X, Y, R, n, $Y^1$ and $Y^2$ are as defined above and wherein $Y^1$ and $Y^2$ can also be included in a ring, and if desired converting a base obtained into a pharmaceutically acceptable acid addition salt or converting a salt obtained into the base.

2. A process according to claim 1, wherein, in the compound prepared, $R^1$ and/or $R^2$ is hydrogen or methyl.

3. A process according to claim 1, wherein, in the compound prepared, $R^1$ and $R^2$ is hydrogen.

4. A process according to claim 1, wherein, in the compound prepared, R, when alkyl, is saturated straight or branched having 1-3 carbon atoms in the chain.

5. A process according to claim 1, wherein, in the compound prepared, R, when cycloalkyl, has 5-6 carbon atoms in the ring.

6. A process according to claim 1, wherein, in the compound prepared, X, Y and $X^1$ are in the meta and/or para positions.

7. A process according to claim 1, wherein, in the compound prepared, halogen X, Y and $X^1$ is bromo, chloro or fluoro in the meta and/or para position.

8. A process according to claim 7, wherein, in the compound prepared, halogen X, Y and $X^1$ is para fluoro.

9. A process according to claim 1, wherein, in the compound prepared, X and/or Y is $CF_3$ in the meta position when being the monosubstituent.

10. A process according to claim 1, wherein, in the compound prepared, X and/or Y is CN in the para position when being the monosubstituent.

11. A process according to claim 1, wherein, in the compound prepared, X and/or Y is methyl in the para position when being the monosubstituent.

12. A process according to claim 9, 10 or 11, wherein, in the compound prepared, R is a saturated alkyl group straight or branched having 1-3 carbon atoms in the chain.

13. A process according to claim 1, wherein, in the compound prepared, n is 0, Z is as defined above, $R^1$ and $R^2$ are both hydrogen, X is para-Cl or para-F and R is an ethyl or an isopropyl group.

14. A process according to claim 13 wherein, in the compound prepared, Z is CO, X is F and R is ethyl.

15. A process according to claim 13, wherein, in the compound prepared, Z is CO, X is F and R is isopropyl.

16. A process according to claim 13, wherein, in the compound prepared, Z is CH(OH), X is F and R is ethyl.

17. A process in accordance with claim 1 for preparing a compound of the general formula Ia

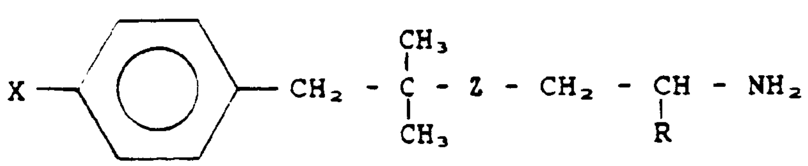

Ia

or a pharmaceutically acceptable salt thereof, wherein Z is CO or CH(OH), X is halogen and R is a straight or branched alkyl group having 1-5 carbon atoms in the chain in racemic or optically active form and when Z is CH(OH) also mixtures of diastereomers or the pure diastereomers, with the proviso that the racemate of the compound, wherein X is Cl, Z is CO and R is methyl is excluded.

18. A process according to claim 17, wherein, in the compound prepared, Z is as defined above, X is Cl or F and R is an ethyl or an isopropyl group.

19. A process according to claim 17 wherein, in the compound prepared, Z is CO, X is F and R is ethyl.

20. A process according to claim 17, wherein, in the compound prepared, Z is CO, X is F and R is isopropyl.

21. A process according to claim 17, wherein, in the compound prepared, Z is CH(OH), X is F and R is ethyl.

22. A process according to one or more of the preceding claims, wherein there is prepared a compound which is the (+)enantiomer.

23. A process according to one or more of the preceding claims, wherein there is prepared a compound which is the (-)enantiomer.

24. A process according to claim 1 wherein there is prepared a compound exhibiting a potentiating effect on central cholinergic responses.

25. A process according to claim 1 wherein there is prepared a compound effective on mental disturbances.

**Patentansprüche**

**Patentansprüche für folgende Vertragsstaaten : AT, BE, CH, DE, FR, GB, IT, LI, LU, NL, SE**

1. Verbindung der allgemeinen Formel I

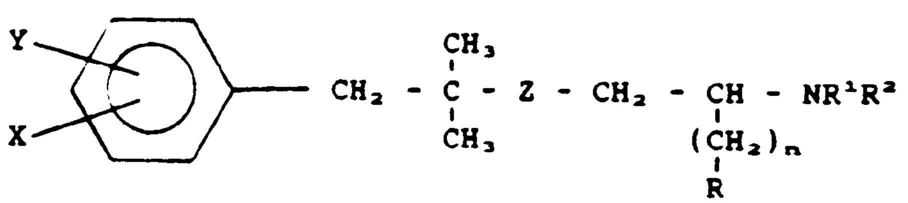

, I

worin n 0 oder 1 ist, Z für CO oder CH(OH) steht, $R^1$ und $R^2$ gleich oder verschieden sind, und, wenn sie gleich sind, Wasserstoff, Methyl, Äthyl oder Propyl bedeuten, und wobei, wenn sie verschieden sind, $R^1$ für Wasserstoff und $R^2$ für Methyl, Äthyl, Propyl oder Isopropyl steht, X und Y Wasserstoff, Halogen, $CF_3$, CN oder eine Alkylgruppe, gerade oder verzweigt mit 1-3 Kohlenstoffatomen, in der ortho-, meta- oder para-Position bedeuten, mit der Maßgabe, daß, wenn X in der ortho-Position steht und etwas anderes als Wasserstoff bedeutet, Y dann etwas anderes als Wasserstoff ist, und, wenn Y in der ortho-Position steht und etwas anderes als Wasserstoff ist, X dann etwas anderes als Wasserstoff ist, R eine gerade oder verzweigte, gesättigte oder ungesättigte 1-6 Kohlenstoff-Alkylgruppe, eine 3-6 Kohlenstoff-Cycloalkylgruppe, eine Phenylgruppe, die unsubstituiert sein kann oder einen Ring-Substituenten $X_1$ tragen kann, wobei $X_1$ vorzugsweise ein Halogen ist, darstellt, oder R Wasserstoff darstellt, in racemischer oder optisch aktiver Form, und, wenn Z CH(OH) ist, auch Mischungen von Diastereomeren oder ein reines Diastereomeres, mit der Maßgabe, daß das Racemat der Verbindung, worin $R^1$ und $R^2$ Wasserstoff bedeuten, X für p-Cl steht, Y Wasserstoff darstellt, Z CO bedeutet, n 0 ist und R Methyl darstellt, ausgeschlossen ist; oder ein pharmazeutisch akzeptables Salz davon.

2. Verbindung nach Anspruch 1, worin $R^1$ und/oder $R^2$ Wasserstoff oder Methyl ist.

3. Verbindung nach Anspruch 1, worin $R^1$ und $R^2$ Wasserstoff sind.

4. Verbindung nach Anspruch 1, worin R, wenn es Alkyl ist, gesättigt, gerade oder verzweigt mit 1-3 Kohlenstoffatomen in der Kette ist.

5. Verbindung nach Anspruch 1, worin R, wenn es Cycloalkyl ist, 5-6 Kohlenstoffatome im Ring hat.

6. Verbindung nach Anspruch 1, worin X, Y und $X^1$ in meta- und/oder para-Position sind.

7. Verbindung nach Anspruch 1, worin Halogen X, Y und $X^1$ Brom, Chlor oder Fluor in meta- und/oder para-Position ist.

8. Verbindung nach Anspruch 7, worin Halogen X, Y und $X^1$ para-Fluor ist.

9. Verbindung nach Anspruch 1, worin X und/oder Y $CF_3$ in meta-Position ist, wenn es der Monosubstituent ist.

10. Verbindung nach Anspruch 1, worin X und/oder Y CN in para-Position ist, wenn es der Monosubstituent ist.

11. Verbindung nach Anspruch 1, worin X und/oder Y Methyl in para-Position ist, wenn es der Monosubstituent ist.

12. Verbindung nach Anspruch 9, 10 oder 11, worin R eine gesättigte Alkylgruppe ist, die gerade oder verzweigt ist und 1-3 Kohlenstoffatome in der Kette hat.

13. Verbindung nach Anspruch 1, worin n 0 ist, Z die obige Bedeutung hat, $R^l$ und $R^2$ beide Wasserstoff bedeuten, X paraCl oder para-F ist und R eine Äthyl- oder eine Isopropylgruppe ist.

14. Verbindung nach Anspruch 13, worin Z für CO steht, X F ist und R Äthyl bedeutet.

15. Verbindung nach Anspruch 13, worin Z für CO steht, X F ist und R Isopropyl bedeutet.

16. Verbindung nach Anspruch 13, worin Z für CH(OH) steht, X F ist und R Äthyl bedeutet.

17. Verbindung der allgemeinen Formel Ia

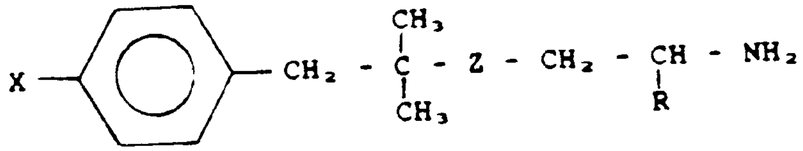

,Ia

oder ein pharmazeutisch akzeptables Salz davon, worin Z für CO oder CH(OH) steht, X Halogen bedeutet und R eine gerade oder verzweigte Alkylgruppe mit 1-5 Kohlenstoffatomen in der Kette in racemischer oder optisch aktiver Form ist, und, wenn Z CH(OH) bedeutet, auch Mischungen von Diastereomeren oder die reinen Diastereomeren, mit der Maßgabe, daß das Racemat der Verbindung, worin X für Cl steht, Z CO ist und R Methyl bedeutet, ausgeschlossen ist.

18. Verbindung nach Anspruch 17, worin Z die obige Bedeutung hat, X für Cl oder F steht und R eine Äthyl- oder eine Isopropylgruppe darstellt.

19. Verbindung nach Anspruch 17, worin Z für CO steht, X F bedeutet und R Äthyl ist.

20. Verbindung nach Anspruch 17, worin Z für CO steht, X F bedeutet und R Isopropyl ist.

21. Verbindung nach Anspruch 17, worin Z für CH(OH) steht, X F bedeutet und R Äthyl ist.

22. Verbindung nach einem oder mehreren der vorhergehenden Ansprüche, welches das (+)Enantiomere ist.

23. Verbindung nach einem oder mehreren der vorhergehenden Ansprüche, welches das (-)Enantiomere ist.

24. Verbindung nach Anspruch 1, welche eine potenzierende Wirkung auf zentral-cholinergische Reaktionen zeigt.

25. Verbindung nach Anspruch 1 mit Wirkung auf Geistesstörungen.

26. Verfahren zur Herstellung einer Verbindung der allgemeinen Formel I

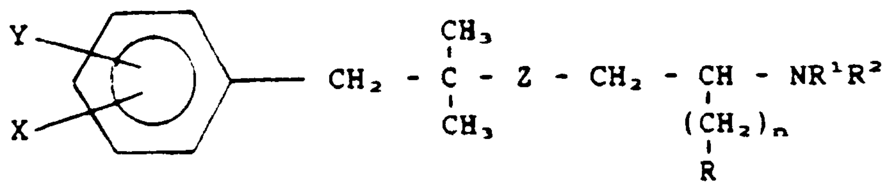

,I

worin Z, X, Y, R, $R^1$, $R^2$ die obige Bedeutung haben, durch

A) Umsetzung einer Verbindung der Formel II

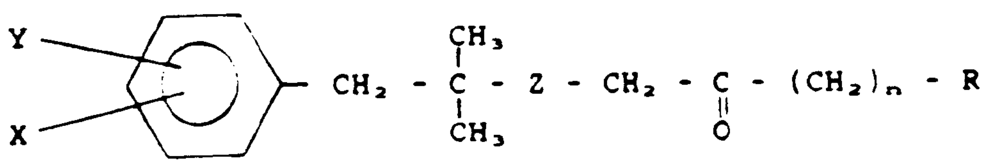

,II

worin Z, X, Y, R und n die obige Bedeutung haben, mit Ammoniak, einem Ammoniumsalz oder einem geeigneten Aminderivat in Anwesenheit eines geeigneten Reduktionsmittels auf schrittweise oder direkte Art, oder

B) Reduktion des entsprechenden Derivats der Formel III

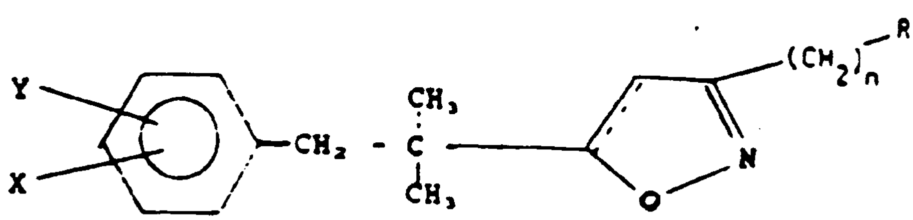

,III

worin X, Y, R und n die obige Bedeutung haben, zu einer Verbindung der allgemeinen Formel I, worin Z, X, Y, R und n die obige Bedeutung haben und $R^1$ und $R^2$ Wasserstoff sind, mittels eines geeigneten Reduktionsverfahrens, oder

C) Überführung eines ungesättigten Ketons der Formel IV

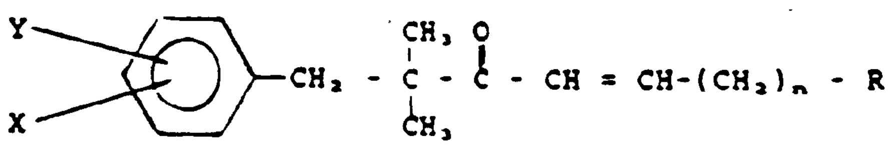

,IV

worin X, Y, R und n die obige Bedeutung haben, in eine Verbindung der allgemeinen Formel I, worin Z für CO steht, X, Y, R, $R^1$, $R^2$ und n die obige Bedeutung haben, durch Zugabe von $HNR^1R^2$, oder

D) Überführung des entsprechenden Derivats der Formel V

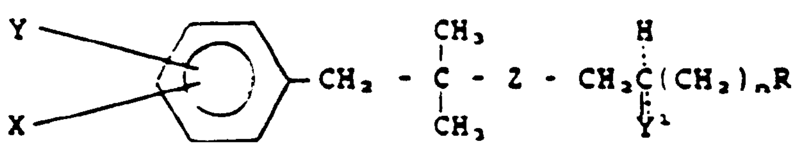

V

worin X, Y, Z, n und R die obige Bedeutung haben und $Y^1$ eine Gruppe darstellt, die in eine $NR^1R^2$-Gruppe umgewandelt werden kann, mittels eines geeigneten hydrolytischen, reduktiven, elektrochemischen, alkylierenden oder anderen bekannten Verfahrens, oder

E) Überführung der entsprechenden Verbindung der allgemeinen Formel VI

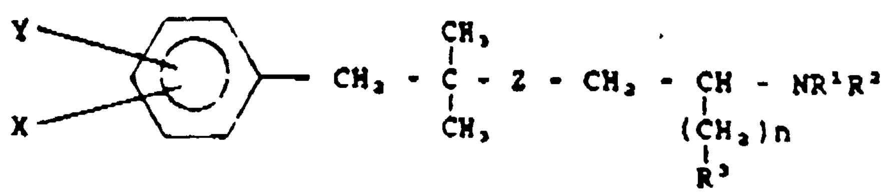

,VI

worin X, Y, Z, n, $R^1$ und $R^2$ die obige Bedeutung haben und $R^3$ eine Gruppe darstellt, die mittels eines geeigneten reduktiven, hydrolytischen oder anderen bekannten Verfahrens in eine wie oben definierte Alkylgruppe R umgewandelt werden kann, oder

F) Reduktion der entsprechenden Verbindung der allgemeinen Formel VII

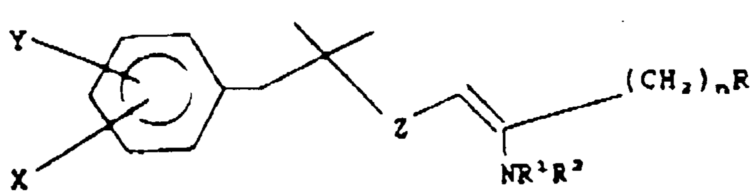

,VII

worin X, Y, Z, R, $R^1$, $R^2$ und n die obige Bedeutung haben, mittels bekannter Verfahren, oder

G) Umsetzung eines Ketons der Formel VIII

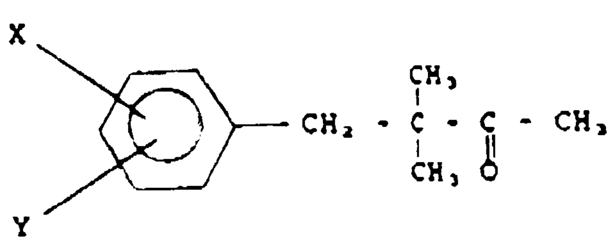

,VIII

worin X und Y die obige Bedeutung haben, mit einem geeigneten Aldehyd $CHO(CH_2)_nR$ und $NHR^1R^2$, worin R, $R^1$, $R^2$ und n die obige Bedeutung haben, oder

H) Auftrennung eines Racemats der Formel I, oder

J) durch Überführung des entsprechenden Derivates der allgemeinen Formel IX

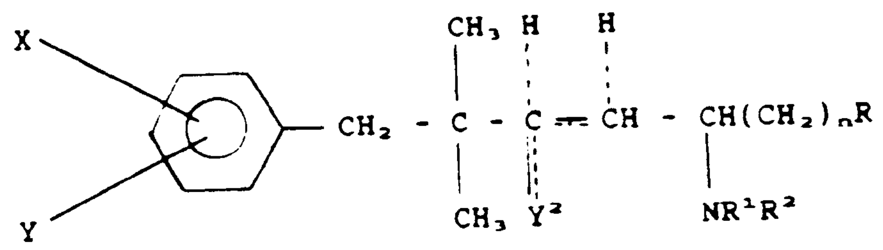 ,IX

worin X, Y, n, R, $R^1$ und $R^2$ die obige Bedeutung haben und $CHY^2$ eine Gruppe darstellt, die in Z, wie oben definiert, umgewandelt werden kann, mittels eines geeigneten hydrolytischen, oxidativen, reduktiven oder anderen bekannten Verfahrens, oder

K) durch Überführung einer Verbindung der allgemeinen Formel X

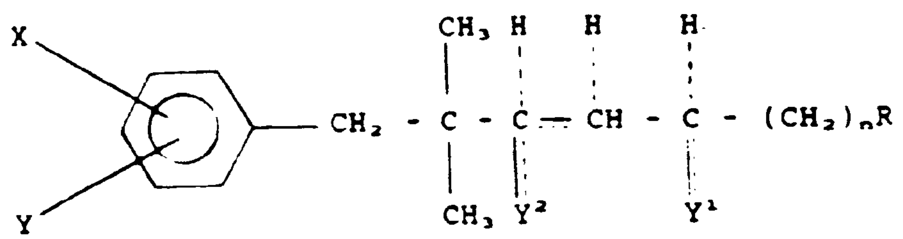 X

in eine Verbindung der allgemeinen Formel I durch Kombination der Verfahren D und J, worin X, Y, R, n, $Y^1$ und $Y^2$ die obige Bedeutung haben und worin $Y^1$ und $Y^2$ auch in einem Ring eingeschlossen sein können, und gewünschtenfalls die Überführung einer erhaltenen Base in ein pharmazeutisch akzeptables Säureadditionssalz oder die Überführung eines erhaltenen Salzes in die Base.

27. Pharmazeutische Präparation, die als aktives Ingrediens eine Verbindung nach einem der Ansprüche 1-25 enthält.

28. Verwendung einer Verbindung der allgemeinen Formel I nach Anspruch 1 oder eines pharmazeutisch akzeptablen Salzes davon für die Herstellung einer pharmazeutischen Formulierung mit Wirkung auf Geistesstörungen.

29. Verwendung einer Verbindung der allgemeinen Formel I nach Anspruch 1 oder eines pharmazeutisch akzeptablen Salzes davon für die Herstellung einer pharmazeutischen Formulierung, die zum Erhalt einer potenzierenden Wirkung auf cholinergische Reaktionen bei einem Patienten mit Geistesstörungen wirksam ist.

**Patentansprüche für folgende Vertragsstaaten : GR, ES**

1. Verfahren zur Herstellung einer Verbindung der allgemeinen Formel I

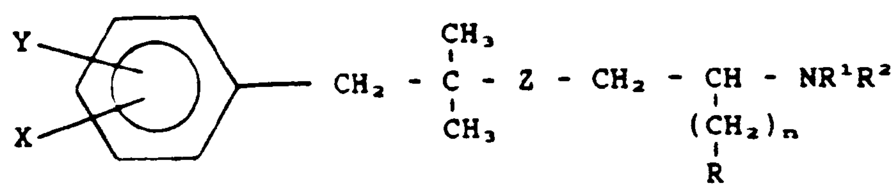 ,I

worin n 0 oder 1 ist, Z für CO oder CH(OH) steht, $R^1$ und $R^2$ gleich oder verschieden sind, und, wenn sie gleich sind, Wasserstoff, Methyl, Äthyl oder Propyl bedeuten, und wobei, wenn sie verschieden sind, $R^1$ für Wasserstoff und $R^2$ für Methyl, Äthyl, Propyl oder Isopropyl steht, X und Y Wasserstoff, Halogen, $CF_3$, CN oder eine Alkylgruppe, gerade oder verzweigt mit 1-3 Kohlenstoffatomen, in der ortho-, meta- oder para-Position bedeuten, mit der Maßgabe, daß, wenn X in der ortho-Position steht und etwas anderes als Wasserstoff bedeutet,

Y dann etwas anderes als Wasserstoff ist, und, wenn Y in der ortho-Position steht und etwas anderes als Wasserstoff ist, X dann etwas anderes als Wasserstoff ist, R eine gerade oder verzweigte, gesättigte oder ungesättigte 1-6 Kohlenstoff-Alkylgruppe, eine 3-6 Kohlenstoff-Cycloalkylgruppe, eine Phenylgruppe, die unsubstituiert sein kann oder einen Ring-Substituenten $X_1$ tragen kann, wobei $X_1$ vorzugsweise ein Halogen ist, darstellt, oder R Wasserstoff darstellt, in racemischer oder optisch aktiver Form, und, wenn Z CH(OH) ist, auch von Mischungen von Diastereomeren oder einem reinen Diastereomeren, mit der Maßgabe, daß das Racemat der Verbindung, worin $R^1$ und $R^2$ Wasserstoff bedeuten, X für p-Cl steht, Y Wasserstoff darstellt, Z CO bedeutet, n 0 ist und R Methyl darstellt, ausgeschlossen ist; oder eines pharmazeutisch akzeptablen Salzes davon, durch

A) Umsetzung einer Verbindung der Formel II

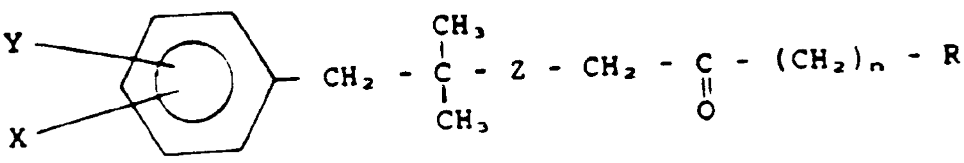

,II

worin Z, X, Y, R und n die obige Bedeutung haben, mit Ammoniak, einem Ammoniumsalz oder einem geeigneten Amin-derivat in Anwesenheit eines geeigneten Reduktionsmittels auf schrittweise oder direkte Art, oder

B) Reduktion des entsprechenden Derivats der Formel III

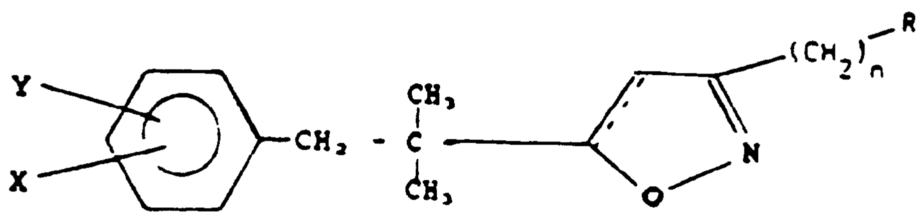

,III

worin X, Y, R und n die obige Bedeutung haben, zu einer Verbindung der allgemeinen Formel I, worin Z, X, Y, R und n die obige Bedeutung haben und $R^1$ und $R^2$ Wasserstoff sind, mittels eines geeigneten Reduktionsverfahrens, oder

C) Überführung eines ungesättigten Ketons der Formel IV

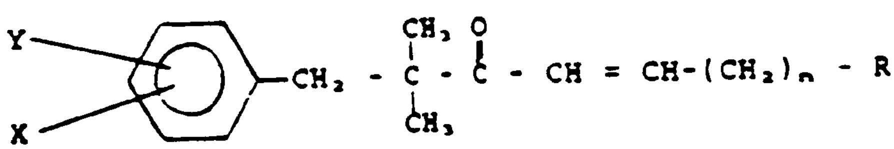

,IV

worin X, Y, R und n die obige Bedeutung haben, in eine Verbindung der allgemeinen Formel I, worin Z für CO steht, X, Y, R, $R^1$, $R^2$ und n die obige Bedeutung haben, durch Zugabe von $HNR^1R^2$, oder

D) Überführung des entsprechenden Derivats der Formel V

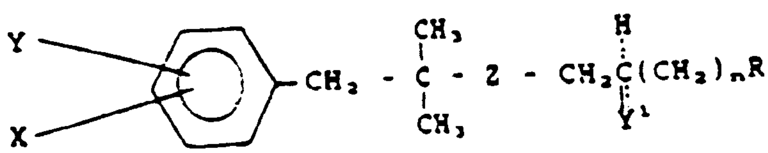

,V

worin X, Y, Z, n und R die obige Bedeutung haben und $Y^1$ eine Gruppe darstellt, die in eine $NR^1R^2$-Gruppe umgewandelt werden kann, mittels eines geeigneten hydrolytischen, reduktiven, elektrochemischen, alkylierenden oder anderen bekannten Verfahrens, oder

E) Überführung der entsprechenden Verbindung der allgemeinen Formel VI

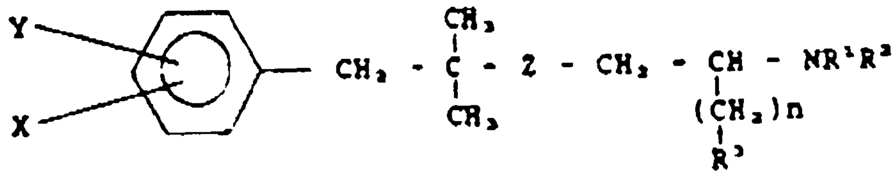

,VI

worin X, Y, Z, n, $R^1$ und $R^2$ die obige Bedeutung haben und $R^3$ eine Gruppe darstellt, die mittels eines geeigneten reduktiven, hydrolytischen oder anderen bekannten Verfahrens in eine wie oben definierte Alkylgruppe R umgewandelt werden kann, oder

F) Reduktion der entsprechenden Verbindung der allgemeinen Formel VII

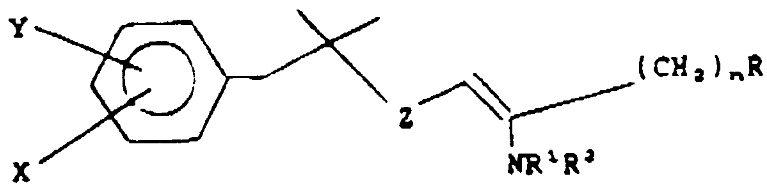

,VII

worin X, Y, Z, R, $R^1$, $R^2$ und n die obige Bedeutung haben, mittels bekannter Verfahren, oder

G) Umsetzung eines Ketons der Formel VIII

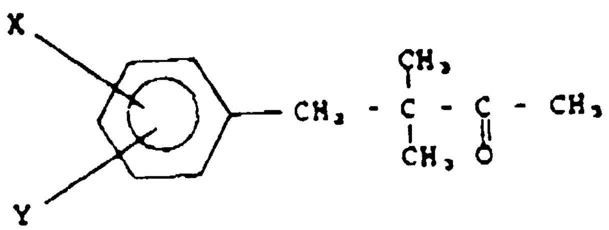

VIII

worin X und Y die obige Bedeutung haben, mit einem geeigneten Aldehyd $CHO(CH_2)_nR$ und $NHR^1R^2$, worin R, $R^1$, $R^2$ und n die obige Bedeutung haben, oder

H) Auftrennung eines Racemats der Formel I, oder

J) durch Überführung des entsprechenden Derivates der allgemeinen Formel IX

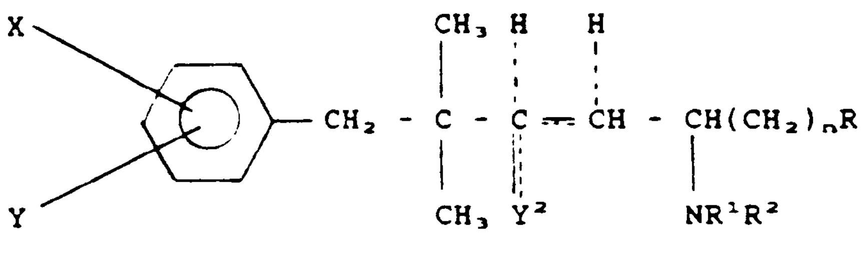

IX

worin X, Y, n, R, $R^1$ und $R^2$ die obige Bedeutung haben und $CHY^2$ eine Gruppe darstellt, die in Z, wie oben definiert, umgewandelt werden kann, mittels eines geeigneten hydrolytischen, oxidativen, reduktiven oder anderen bekannten Verfahrens, oder

K) durch Überführung einer Verbindung der allgemeinen Formel X

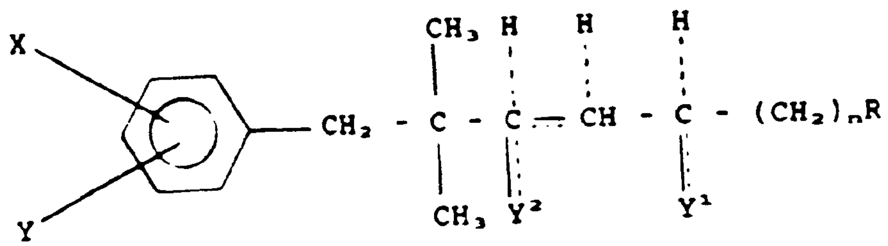

X

in eine Verbindung der allgemeinen Formel I durch Rombination der Verfahren D und J, worin X, Y, R, n, $Y^1$ und $Y^2$ die obige Bedeutung haben und worin $Y^1$und $Y^2$auch in einem Ring eingeschlossen sein können, und gewünschtenfalls die Überführung einer erhaltenen Base in ein pharmazeutisch akzeptables Säureadditionssalz oder die Überführung eines erhaltenen Salzes in die Base.

2. Verfahren nach Anspruch 1, worin in der hergestellten Verbindung $R^1$ und/oder $R^2$ Wasserstoff oder Methyl ist.

3. Verfahren nach Anspruch 1, worin in der hergestellten Verbindung $R^1$ und $R^2$ Wasserstoff sind.

4. Verfahren nach Anspruch 1, worin in der hergestellten Verbindung R, wenn es Alkyl ist, gesättigt, gerade oder verzweigt mit 1-3 Kohlenstoffatomen in der Kette ist.

5. Verfahren nach Anspruch 1, worin in der hergestellten verbindung R, wenn es Cycloalkyl ist, 5-6 Kohlenstoffatome im Ring hat.

6. Verfahren nach Anspruch 1, worin in der hergestellten Verbindung X, Y und $X^1$ in meta- und/oder para-Position sind.

7. Verfahren nach Anspruch 1, worin in der hergestellten Verbindung Halogen X, Y und $X^1$ Brom, Chlor oder Fluor in meta- und/oder para-Position ist.

8. Verfahren nach Anspruch 7, worin in der hergestellten Verbindung Halogen X, Y und $X^1$ para-Fluor ist.

9. Verfahren nach Anspruch 1, worin in der hergestellten Verbindung X und/oder Y $CF_3$ in meta-Position ist, wenn es der Monosubstituent ist.

10. Verfahren nach Anspruch 1, worin in der hergestellten Verbindung X und/oder Y CN in para-Position ist, wenn es der Monosubstituent ist.

11. Verfahren nach Anspruch 1, worin in der hergestellten Verbindung X und/oder Y Methyl in para-Position ist, wenn es der Monosubstituent ist.

12. Verfahren nach Anspruch 9, 10 oder 11, worin in der hergestellten Verbindung R eine gesättigte Alkylgruppe ist, die gerade oder verzweigt ist und 1-3 Kohlenstoffatome in der Kette hat.

13. Verfahren nach Anspruch 1, worin in der hergestellten Verbindung n 0 ist, Z die obige Bedeutung hat, $R^1$ und $R^2$ beide Wasserstoff bedeuten, X para-Cl oder para-F ist und R eine Äthyl- oder eine Isopropylgruppe ist.

14. Verfahren nach Anspruch 13, worin in der hergestellten Verbindung Z für CO steht, X F ist und R Äthyl bedeutet.

15. Verfahren nach Anspruch 13, worin in der hergestellten Verbindung Z für CO steht, X F ist und R Isopropyl bedeutet.

16. Verfahren nach Anspruch 13, worin in der hergestellten Verbindung Z für CH(OH) steht, X F ist und R Äthyl bedeutet.

17. Verfahren nach Anspruch 1 zur herstellung einer Verbindung der allgemeinen Formel Ia

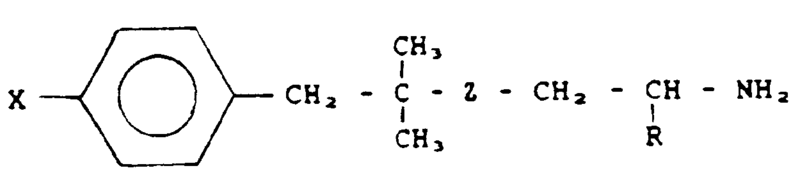

Ia

oder eines pharmazeutisch akzeptablen Salzes davon, worin Z für CO oder CH(OH) steht, X Halogen bedeutet und R eine gerade oder verzweigte Alkylgruppe mit 1-5 Kohlenstoffatomen in der Kette ist, in racemischer oder optisch aktiver Form, und, wenn Z CH(OH) bedeutet, auch von Mischungen von Diastereomeren oder von reinen Diastereomeren, mit der Maßgabe, daß das Racemat der Verbindung, worin X für Cl steht, Z CO ist und R Methyl bedeutet, ausgeschlossen ist.

18. Verfahren nach Anspruch 17, worin in der hergestellten Verbindung Z die obige Bedeutung hat, X für Cl oder F steht und R eine Äthyl- oder eine Isopropylgruppe darstellt.

19. Verfahren nach Anspruch 17, worin in der hergestellten Verbindung Z für CO steht, X F bedeutet und R Äthyl ist.

20. Verfahren nach Anspruch 17, worin in der hergestellten Verbindung Z für CO steht, X F bedeutet und R Isopropyl ist.

21. Verfahren nach Anspruch 17, worin in der hergestellten Verbindung Z für CH(OH) steht, X F bedeutet und R Äthyl ist.

22. Verfahren nach einem oder mehreren der vorhergehenden Ansprüche, worin eine Verbindung hergestellt wird, die das (+)Enantiomere ist.

23. Verfahren nach einem oder mehreren der vorhergehenden Ansprüche, worin eine Verbindung hergestellt wird, die das (-)Enantiomere ist.

24. Verfahren nach Anspruch 1, worin eine Verbindung hergestellt wird, welche eine potenzierende Wirkung auf zentrale cholinergische Reaktionen zeigt.

25. Verfahren nach Anspruch 1, worin eine Verbindung mit Wirkung auf Geistesstörungen hergestellt wird.

## Revendications

### Revendications pour les Etats contractants suivants : AT, BE, CH, DE, FR, GB, IT, LI, LU, NL, SE

1. Composé de formule générale I.

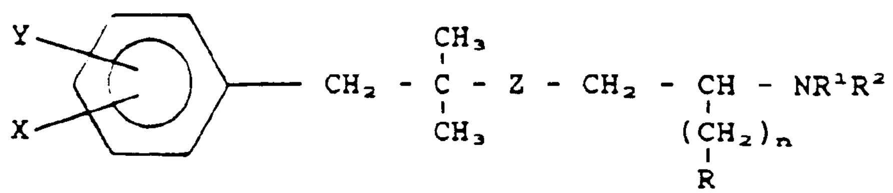

I

dans lequel n vaut 0 ou 1, Z est CO ou CH(OH), $R^1$ et $R^2$ sont identiques ou différents et, quand ils sont identiques, sont l'hydrogène, le méthyle, l'éthyle ou le propyle et, quand ils sont différents, $R^1$ est l'hydrogène et $R^2$ est le méthyle, l'éthyle, le propyle ou l'isopropyle, X et Y représentent l'hydrogène, un halogène, $CF_3$, CN ou un groupe alkyle, à chaîne droite ou ramifiée ayant 1 à 3 atomes de carbone, en position ortho, méta ou para pourvu que quand X est en position ortho et autre que l'hydrogène, alors Y est autre que l'hydrogène, et quand Y est en position ortho et autre que l'hydrogène, alors X est autre que l'hydrogène, R représente un groupe alkyle à chaîne droite ou ramifiée, saturée ou insaturée, ayant de 1 à 6 atomes de carbone, un groupe cycloalkyle de 3 à 6 atomes de carbone, un groupe phényle, qui peut être non-substitué ou porter un substituant de cycle $X^1$, $X^1$ étant de préférence un halogène, ou R représente l'hydrogène, sous forme racémique ou optiquement active et quand Z est CH(OH) également des mélanges de diastéréoisomères ou un diastéréoisomère pur, à condition que le racémate du composé, dans lequel $R^1$ et $R^2$ sont l'hydrogène, X est p-Cl, Y est l'hydrogène, Z est CO, n vaut 0 et R est le méthyle, soit exclu; ou son sel pharmaceutiquement acceptable.

2. Composé selon la revendication 1, dans lequel $R^1$ et/ou $R^2$ est l'hydrogène ou le méthyle.

3. Composé selon la revendication 1, dans lequel $R^1$ et $R^2$ sont l'hydrogène.

4. Composé selon la revendication 1, dans lequel R, quand il est un alkyle, est une chaîne droite ou ramifiée saturée ayant 1 à 3 atomes de carbone dans la chaîne.

5. Composé selon la revendication 1, dans lequel R, quand il est un cycloalkyle, a 5 à 6 atomes de carbone dans le cycle.

6. Composé selon la revendication 1, dans lequel X, Y et $X^1$ sont en position méta et/ou para.

7. Composé selon la revendication 1, dans lequel les halogènes X, Y et $X^1$ sont le bromo, le chloro ou le fluoro en position méta et/ou para.

8. Composé selon la revendication 7, dans lequel les halogènes X, Y et $X^1$ sont le para-fluoro.

9. Composé selon la revendication 1, dans lequel X et/ou Y est $CF_3$ en position méta quand il est le monosubstituant.

10. Composé selon la revendication 1, dans lequel X et/ou Y est CN en position para quand il est le monosubstituant.

11. Composé selon la revendication 1, dans lequel X et/ou Y est le méthyle en position para quand il est le monosubstituant.

12. Composé selon la revendication 9, 10 ou 11, dans lequel R est un groupe alkyle saturé à chaîne droite ou ramifiée ayant 1 à 3 atomes de carbone dans la chaîne.

13. Composé selon la revendication 1, dans lequel n vaut 0, Z est défini comme ci-dessus, $R^1$ et $R^2$ sont tous deux de l'hydrogène, X est para-Cl ou para-F et R est un groupe éthyle ou isopropyle.

14. Composé selon la revendication 13, dans lequel Z est CO, X est F et R est l'éthyle.

15. Composé selon la revendication 13, dans lequel Z est CO, X est F et R est l'isopropyle.

16. Composé selon la revendication 13, dans lequel Z est CH(OH), X est F et R est l'éthyle.

17, Composé de formule générale Ia

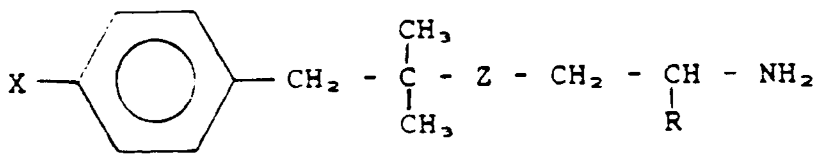

Ia

ou son sel pharmaceutiquement acceptable, dans lequel Z est CO ou CH(OH), X est un halogène et R est un groupe alkyle à chaîne droite ou ramifiée ayant 1 à 5 atomes de carbone dans la chaîne sous forme racémique ou optiquement active et quand Z est CH(OH) également des mélanges de diastéréoisomères ou les diastéréoisomères purs, à condition que le racémate du composé, dans lequel X est Cl, Z est CO et R est le méthyle, soit exclu.

18. Composé selon la revendication 17, dans lequel Z est défini comme ci-dessus, X est Cl ou F et R est un groupe éthyle ou isopropyle.

19. Composé selon la revendication 17, dans lequel Z est CO, X est F et R est l'éthyle.

20. Composé selon la revendication 17, dans lequel Z est CO, X est F et R est l'isopropyle,

21. Composé selon la revendication 17, dans lequel Z est CH(OH), X est F et R est l'éthyle.

22. Composé selon l'une ou plusieurs des revendications précédentes, qui est le (+)énantiomère.

23. Composé selon l'une ou plusieurs des revendications précédentes, qui est le (-)énantiomère.

24. Composé selon la revendication 1 montrant un effet de potentialisation sur les réponses cholinergiques centrales.

25. Composé selon la revendication 1 efficace sur les troubles mentaux.

26. Procédé pour la préparation d'un composé de formule générale I

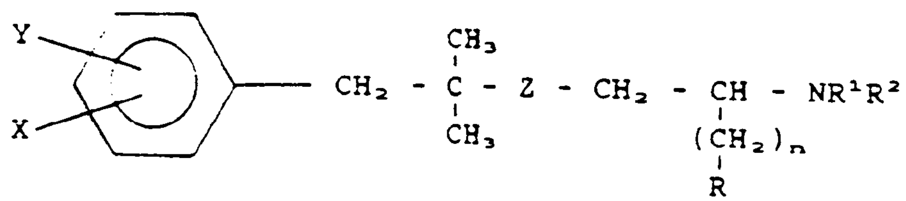

I

dans lequel Z, X, Y, R, $R^1$, $R^2$ et n sont définis comme ci-dessus, par

A) la mise en réaction d'un composé de formule II

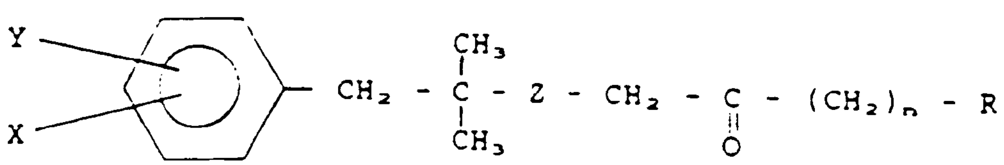

II

dans lequel Z, X, Y, R et n sont définis comme ci-dessus, avec l'ammoniac, un sel d'ammonium ou un dérivé amine approprié en présence d'un agent réducteur approprié de façon progressive ou directe, ou

B) réduction du dérivé correspondant de formule III

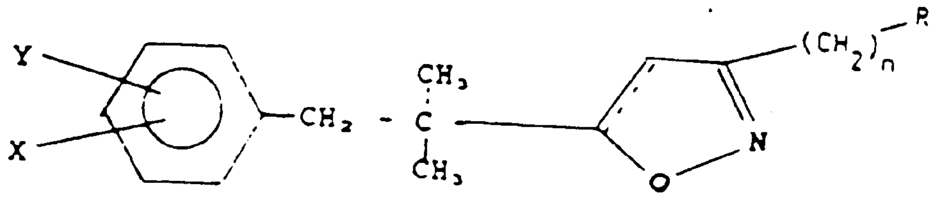

III

dans lequel X, Y, R et n sont définis comme ci-dessus, en un composé de formule générale I dans lequel Z, X, Y, R et n sont définis comme ci-dessus, et $R^1$ et $R^2$ sont l'hydrogène, par un procédé de réduction approprié, ou

C) conversion d'une cétone insaturée de formule IV

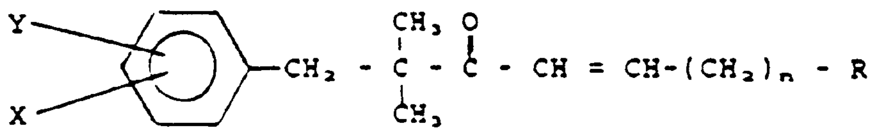

IV

dans laquelle X, Y, R et n sont définis comme ci-dessus, en un composé de formule générale I dans lequel Z est CO, X, Y, R, $R^1$, $R^2$ et n sont définis comme ci-dessus, par addition de $HNR^1R^2$, ou

D) conversion des dérivés correspondants de formule V

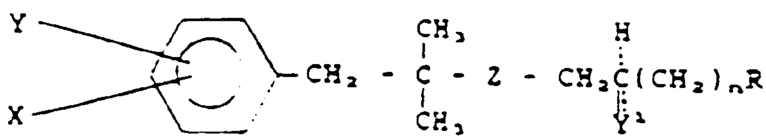

V

dans lequel X, Y, Z, n et R sont définis comme ci-dessus et $Y^1$ représente un groupe qui peut être transformé en un groupe $NR^1R^2$ par un procédé approprié hydrolytique, de réduction, électrochimique, d'alkylation ou un autre procédé connu, ou

E) conversion du composé correspondant de formule générale VI

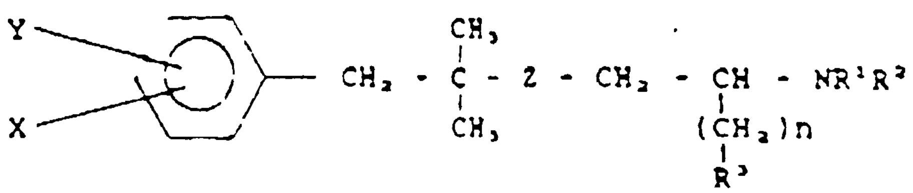

VI

dans lequel X, Y, Z, n, $R^1$ et $R^2$ sont définis comme ci-dessus et $R^3$ représente un groupe qui peut être transformé en un groupe alkyle R défini comme ci-dessus par un procédé de réduction, hydrolytique ou un autre procédé connu, ou

F) réduction du composé correspondant de formule générale VII

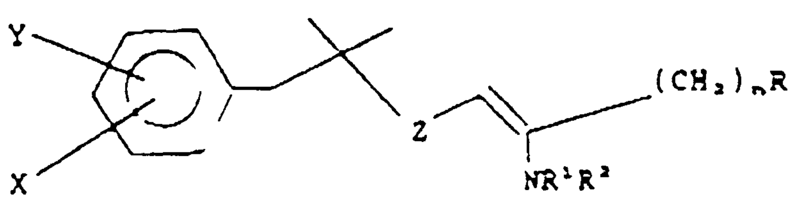

VII

dans lequel X, Y, Z, R, $R^1$, $R^2$ et n sont définis comme ci-dessus, par des procédés connus, ou

G) mise en réaction d'une cétone de formule VIII

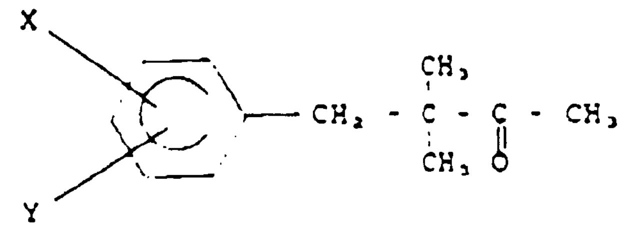

VIII

dans laquelle X et Y sont définis comme ci-dessus avec un aldéhyde approprié $CHO(CH_2)_nR$ et $NHR^1R^2$ dans lesquels R, $R^1$, $R^2$ et n sont définis comme ci-dessus, ou

H) résolution d'un racémate de formule I, ou

J) par conversion des dérivés correspondants de formule générale IX

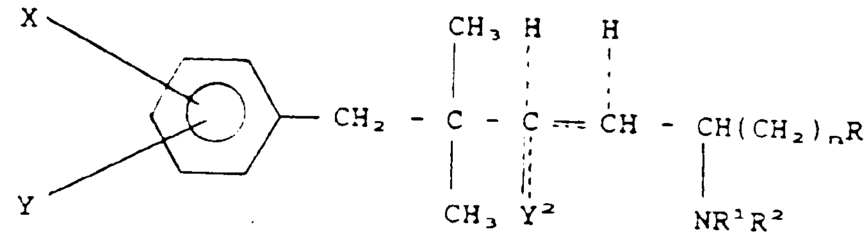

IX

dans lesquels X, Y, n, R, $R^1$ et $R^2$ sont définis comme ci-dessus et $CHY^2$ représente un groupe qui peut être transformé en Z défini comme ci-dessus par un procédé approprié d'hydrolyse, d'oxydation, de réduction ou autres procédés connus, ou

K) par conversion d'un procédé de formule générale X

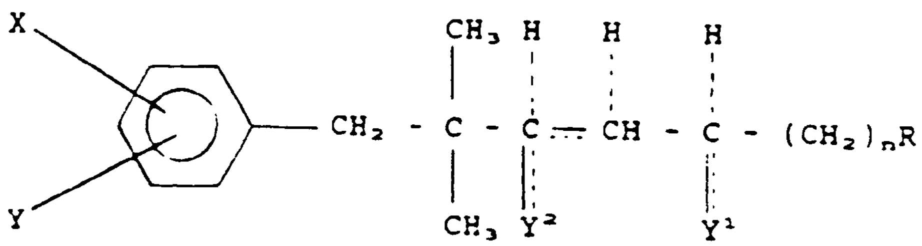

X

en un composé de formule générale I par la combinaison des procédés D et J, dans lequel X, Y, R, n, $Y^1$ et $Y^2$ sont définis comme ci-dessus et dans lequel $Y^1$ et $Y^2$ peuvent également être inclus dans un cycle, et si souhaité conversion d'une base obtenue dans un sel d'addition acide acceptable pharmaceutiquement ou conversion d'un sel obtenu dans la base.

27. Préparation pharmaceutique contenant comme principe actif un composé selon l'une quelconque des revendications 1 à 25.

28. Utilisation d'un composé avec la formule générale I selon la revendication 1 ou son sel pharmaceutiquement acceptable pour la préparation d'une formulation pharmaceutique avec effet sur les troubles mentaux.

29. Utilisation d'un composé de formule générale I selon la revendication 1 ou son sel pharmaceutiquement acceptable pour la préparation d'une formulation pharmaceutique avec effet potentialisant sur les réponses cholinergiques chez un patient ayant des troubles mentaux.

**Revendications pour les Etats contractants suivants : GR, ES**

1. Procédé pour la préparation d'un composé de formule générale I.

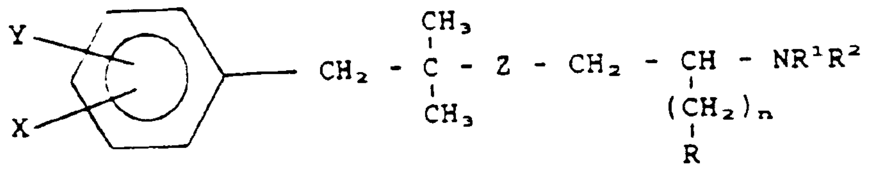

I

dans lequel n vaut 0 ou 1, Z est CO ou CH(OH), $R^1$ et $R^2$ sont identiques ou différents et, quand ils sont identiques, sont l'hydrogène, le méthyle, l'éthyle ou le propyle et, quand ils sont différents, $R^1$ est l'hydrogène et $R^2$ est le méthyle, l'éthyle, le propyle ou l'isopropyle, X et Y représentent l'hydrogène, un halogène, $CF_3$, CN ou un groupe alkyle, à chaîne droite ou ramifiée ayant 1 à 3 atomes de carbone, en position ortho, méta ou para pourvu que quand X est en position ortho et autre que l'hydrogène, alors Y est autre que l'hydrogène, et quand Y est en position ortho et autre que l'hydrogène, alors X est autre que l'hydrogène, R représente un groupe alkyle à chaîne droite ou ramifiée, saturée ou insaturée, ayant de 1 à 6 atomes de carbone, un groupe cycloalkyle de 3 à 6 atomes de carbone, un groupe phényle, qui peut être non-substitué ou porter un substituant de cycle $X^1$, $X^1$ étant de préférence un halogène, ou R représente l'hydrogène, sous forme racémique ou optiquement active et quand Z est CH(OH) également des mélanges de diastéréoisomères ou un diastéréoisomère pur, à condition que le racémate du composé, dans lequel $R^1$ et $R^2$ sont l'hydrogène, X est p-Cl, Y est l'hydrogène, Z est CO, n vaut 0 et R est le méthyle, soit exclu; ou son sel pharmaceutiquement acceptable, par

A) la mise en réaction d'un composé de formule II

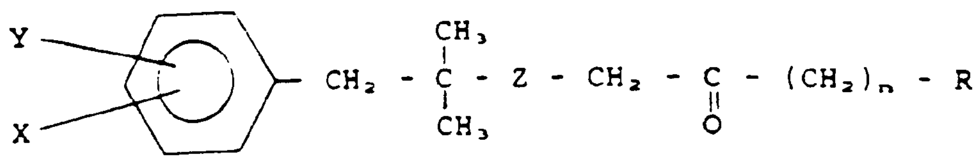

II

dans lequel Z, X, Y, R et n sont définis comme ci-dessus, avec l'ammoniac, un sel d'ammonium ou un dérivé amine approprié en présence d'un agent réducteur approprié de façon pas-à-pas ou directe, ou

B) réduction du dérivé correspondant à la formule III

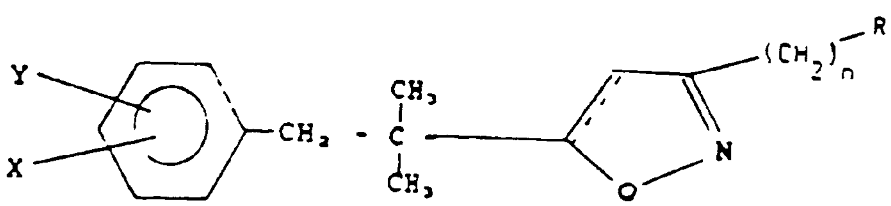

III

dans lequel X, Y, R et n sont définis comme ci-dessus, en un composé de formule générale I dans lequel Z, X, Y, R et n sont définis comme ci-dessus, et $R^1$ et $R^2$ sont l'hydrogène, par un procédé de réduction approprié, ou

C) conversion d'une cétone insaturée de formule IV

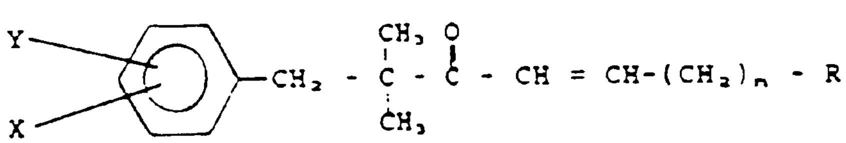

IV

dans laquelle X, Y, R et n sont définis comme ci-dessus, en un composé de formule générale I dans lequel Z est CO, X, Y, R, $R^1$, $R^2$ et n sont définis comme ci-dessus, par addition de $HNR^1R^2$, ou

D) conversion des dérivés correspondant à la formule V

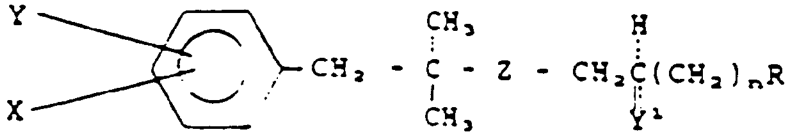

V

dans lequel X, Y, Z, n et R sont définis comme ci-dessus et $Y^1$ représente un groupe qui peut être transformé en un groupe $NR^1R^2$ par un procédé approprié hydrolytique, de réduction, électrochimique, d'alkylation ou un autre procédé connu, ou

E) conversion du composé correspondant de formule générale VI

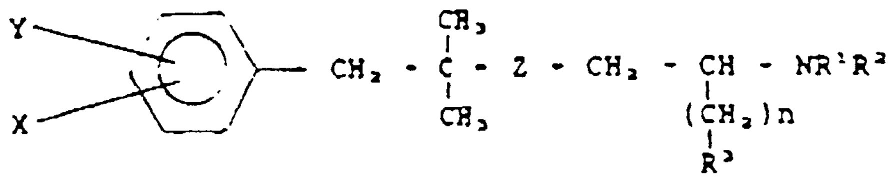

VI

dans lequel X, Y, Z, n, $R^1$ et $R^2$ sont définis comme ci-dessus et $R^3$ représente un groupe qui peut être transformé en un groupe alkyle R défini comme ci-dessus par un procédé de réduction, hydrolytique ou un autre procédé connu, ou

F) réduction du composé correspondant de formule générale VII

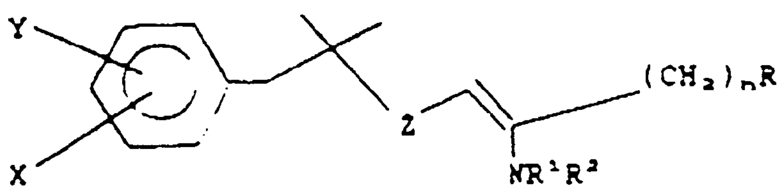

VII

dans lequel X, Y, Z, R, $R^1$, $R^2$ et n sont définis comme ci-dessus, par des procédés connus, ou

G) mise en réaction d'une cétone de formule VIII

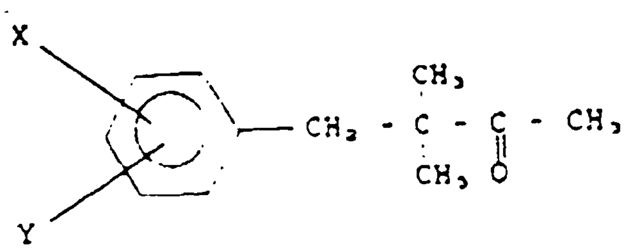

VIII

dans laquelle X et Y sont définis comme ci-dessus avec un aldéhyde approprié $CHO(CH_2)_nR$ et $NHR^1R^2$ dans lesquels R, $R^1$, $R^2$ et n sont définis comme ci-dessus, ou

H) résolution d'un racémate de formule I, ou

J) par conversion des dérivés correspondant à la formule générale IX

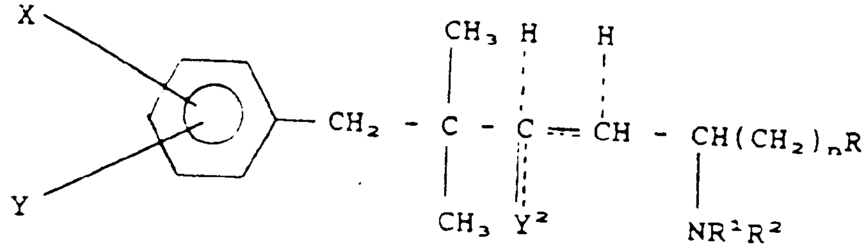

IX

dans lesquels X, Y, n, R, $R^1$ et $R^2$ sont définis comme ci-dessus et $CHY^2$ représente un groupe qui peut être transformé en Z défini comme ci-dessus par une hydrolyse, une oxydation, une réduction appropriée ou par d'autres procédés connus, ou

K) par conversion d'un composé de formule générale X

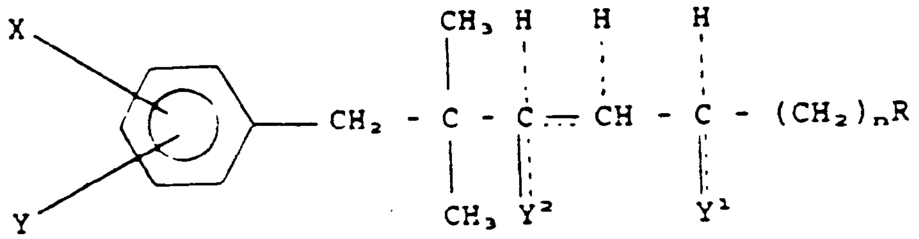

X

en un composé de formule générale I par la combinaison des procédés D et J, dans lequel X, Y, R, n, $Y^1$ et $Y^2$ sont définis comme ci-dessus et dans lequel $Y^1$ et $Y^2$ peuvent également être inclus dans un cycle, et si souhaité conversion d'une base obtenue dans un sel d'addition acide acceptable pharmaceutiquement ou conversion d'un sel obtenu dans la base.

2. Procédé selon la revendication 1, dans lequel, dans le composé préparé, $R^1$ et/ou $R^2$ est l'hydrogène ou le méthyle.

3. Procédé selon la revendication 1, dans lequel, dans le composé préparé, $R^1$ et $R^2$ sont l'hydrogène.

4. Procédé selon la revendication 1, dans lequel, dans le composé préparé, R, quand il est alkyle, est une chaîne droite ou ramifiée saturée ayant 1 à 3 atomes de carbone dans la chaîne.

5. Procédé selon la revendication 1, dans lequel, dans le composé préparé, R, quand il est un cycloalkyle, a 5 à 6 atomes de carbone dans le cycle.

6. Procédé selon la revendication 1, dans lequel, dans le composé préparé, X, Y et $X^1$ sont en position méta et/ou para.

7. Procédé selon la revendication 1, dans lequel, dans le composé préparé, les halogènes X, Y et $X^1$ sont le bromo, le chloro ou le fluoro en position méta et/ou para.

8. Procédé selon la revendication 7, dans lequel, dans le composé préparé, les halogènes X, Y et $X^1$ sont le para-fluoro.

9. Procédé selon la revendication 1, dans lequel, dans le composé préparé, X et/ou Y est $CF_3$ en position méta quand il est le monosubstituant.

10. Procédé selon la revendication 1, dans lequel, dans le composé préparé, X et/ou Y est CN en position para quand il est le monosubstituant.

11. Procédé selon la revendication 1, dans lequel, dans le composé préparé, X et/ou Y est le méthyle en position para quand il est le monosubstituant.

12. Procédé selon la revendication 9, 10 ou 11, dans lequel, dans le composé préparé, R est un groupe alkyle saturé à chaîne droite ou ramifiée ayant 1 à 3 atomes de carbone dans la chaîne.

13. Procédé selon la revendication 1, dans lequel, dans le composé préparé, n vaut 0, Z est défini comme ci-dessus, $R^1$ et $R^2$ sont tous deux de l'hydrogène, X est para-Cl ou para-F et R est un groupe éthyle ou isopropyle.

14. Procédé selon la revendication 13, dans lequel, dans le composé préparé, Z est CO, X est F et R est l'éthyle.

15. Procédé selon la revendication 13, dans lequel, dans le composé préparé, Z est CO, X est F et R est l'isopropyle.

16. Procédé selon la revendication 13, dans lequel, dans le composé préparé, Z est CH(OH), X est F et R est l'éthyle.

17. Procédé selon la revendication 1 pour préparer un composé de formule générale Ia

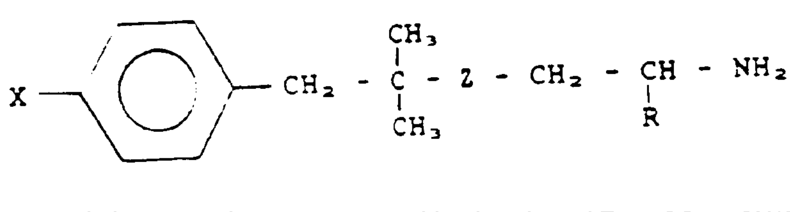

Ia

ou son sel pharmaceutiquement acceptable, dans lequel Z est CO ou CH(OH), X est un halogène et R est un groupe alkyle à chaîne droite ou ramifiée ayant 1 à 5 atomes de carbone dans la chaîne sous forme racémique

ou optiquement active et quand Z est CH(OH) également des mélanges de diastéréoisomères ou les diastéréoisomères purs, à condition que le racémate du composé, dans lequel X est Cl, Z est CO et R est le méthyle, soit exclu.

18. Procédé selon la revendication 17, dans lequel, dans le composé préparé, Z est défini comme ci-dessus, X est Cl ou F et R est un groupe éthyle ou isopropyle.

19. Procédé selon la revendication 17, dans lequel, dans le composé préparé, Z est CO, X est F et R est l'éthyle.

20. Procédé selon la revendication 17, dans lequel, dans le composé préparé, Z est CO, X est F et R est l'isopropyle.

21. Procédé selon la revendication 17, dans lequel, dans le composé préparé, Z est CH(OH), X est F et R est l'éthyle.

22. Procédé selon l'une ou plusieurs des revendications précédentes, dans lequel il est préparé un composé qui est le (+)énantiomère.

23. Procédé selon l'une ou plusieurs des revendications précédentes, dans lequel il est préparé un composé qui est le (-)énantiomère.

24. Procédé selon la revendication 1, dans lequel il est préparé un composé montrant un effet de potentialisation sur les réponses cholinergiques centrales.

25. Procédé selon la revendication 1, dans lequel il est préparé un composé efficace sur les troubles mentaux.